# NEW EUROPEAN PATENT SPECIFICATION

(11) **EP 1 448 566 B3**
(45) Date of publication of this specification: **08.04.2009**
(45) Mention of the grant of the patent: 15.03.2006
(21) Application number: 02789725.5
(22) Date of filing: 13.11.2002
(51) Int. Cl.: C07D 491/04, C07D 471/04, C07D 519/00, C07D 513/04, A61K 31/4353, A61K 31/55, A61P 29/00, A61P 1/00, A61P 3/10, C07D 313/00, C07D 221/00, C07D 223/00

(54) **CHEMOKINE RECEPTOR ANTAGONISTS AND METHODS OF USE THEREOF**
CHEMOKINE REZEPTOR ANTAGONISTEN UND METHODEN ZU DEREN ANWENDUNG
ANTAGONISTES DES RECEPTEURS DE LA CHIMIOKINE ET METHODES D'UTILISATION DE CES ANTAGONISTES

(30) Priority: 21.11.2001 US 989086
(43) Date of publication of application: 25.08.2004
(60) Divisional application: 06004401.3
(73) Proprietor: MILLENNIUM PHARMACEUTICALS, INC., Cambridge, MA 02139 (US); KYOWA HAKKO KOGYO CO., LTD., Chiyoda-ku, Tokyo 100 (JP)
(72) Inventor: LULY, Jay, R., Wellesley, MA 02481 (US); NAKASATO, Yoshisuke, Susono-shi, Shizuoka (JP); OHSHIMA, Etsuo, Nagareyama-shi Chiba (JP); HARRIMAN, Geraldine, C., B., Charlestown, RI 02813 (US); CARSON, Kenneth, G., Needham, MA 02194 (US); GHOSH, Shomir, Brookline, MA 02446 (US); ELDER, Amy, M., Arlington, MA 02474 (US); MATTIA, Karen, M., Marlborough, MA 01752 (US)
(74) Representative: Keen, Celia Mary
(86) International application number: PCT/US2002/036953
(87) International publication number: WO 2003/045942

(56) References cited:
- WO-A-01/09138
- WO-A-96/31469
- WO-A-99/37651

## Description

### RELATED APPLICATIONS

This application is a continuation-in-part of U.S. Application No. 09/989,086, filed November 21, 2001, which is a continuation-in-part of U.S. Application No. 09/627,886, filed July 28, 2000, which is a continuation-in-part of U.S. Application No. 09/362,837, filed July 28,1999, which is a continuation-in-part of U.S. Application No. 09/235,102, filed January 21,1999, which is a continuation-in-part of U.S. Application No. 09/148,823, filed September 4, 1998,

### BACKGROUND OF THE INVENTION

Chemoattractant cytokines or chemokines are a family of proinflammatory mediators that promote recruitment and activation of multiple lineages of leukocytes and lymphocytes. They can be released by many kinds of tissue cells after activation. Continuous release of chemokines at sites of inflammation mediates the ongoing migration of effector cells in chronic inflammation. The chemokines characterized to date are related in primary structure. They share four conserved cysteines, which.form disulfide bonds. Based upon this conserved cysteine motif, the family is divided into two main branches, designated as the C-X-C chemokines (α-chemokines), and the C-C chemokines (β-chemokines), in which the first two conserved cysteines are separated by an intervening residue, or adjacent respectively (Baggiolini, M. and Dahinden, C. A., Immunology Today, 15:127-133 (1994)).

The C-X-C chemokines include a number of potent chemoattractants and activators of neutrophils, such as interleukin 8 (IL-8), PF4 and neutrophil-activating peptide-2 (NAP-2). The C-C chemokines include RANTES (Regulated on Activation, Normal T Expressed and Secreted), the macrophage inflammatory proteins 1α and 1β (MIP-1α and MIP-1β), eotaxin and human monocyte chemotactic proteins 1-3 (MCP-1, MCP-2, MCP-3), which have been characterized as chemoattractants and activators of monocytes or lymphocytes but do not appear to be chemoattractants for neutrophils. Chemokines, such as RANTES and MIP-1α, have been implicated in a wide range of human acute and chronic inflammatory diseases including respiratory diseases, such as asthma and allergic disorders.

The chemokine receptors are members of a superfamily of G protein-coupled receptors (GPCR) which share structural features that reflect a common mechanism of action of signal transduction (Gerard, C. and Gerard, N.P., Annu Rev. Immunol., 12:775-808 (1994); Gerard, C. and Gerard, N. P., Curr. Opin. Immunol., 6:140-145 (1994)). Conserved features include seven hydrophobic domains spanning the piasma membrane, which are connected by hydrophilic extracellular and intracellular loops. The majority of the primary sequence homology occurs in the hydrophobic transmembrane regions with the hydrophilic regions being more diverse. The first receptor for the C-C chemokines that was cloned and expressed binds the chemokines MIP-1α and RANTES. Accordingly, this MIP-1α/RANTES receptor was designated C-C chemokine receptor 1 (also referred to as CCR-1; Neote, K., et al., Cell, 72:415-425 (1993); Horuk, R et al., WO 94/11504, May 26, 1994; Gao, J.-I. et al., J. Exp. Med., 177:1421-1427 (1993)). Three receptors have been characterized which bind and/or signal in response to RANTES: CCR3 mediates binding and signaling of chemokines including eotaxin, RANTES, and MCP-3 (Ponath et al., J. Exp. Med., 183:2437 (1996)), CCR4 binds chemokines including RANTES, MIP-1α, and MCP-1 (Power, et al., J. Biol. Chem., 270:19495 (1995)), and CCR5 binds chemokines including MIP·1α, RANTES, and MIP-1β (Samson, et al., Biochem. 35:3362-3367 (1996)). RANTES is a chemotactic chemokine for a variety of cell types, including monocytes, eosinophils, and a subset of T-cells. The responses of these different cells may not all be mediated by the same receptor, and it is possible that the receptors CCR1, CCR4 and CCR5 will show some selectivity in receptor distribution and function between leukocyte types, as has already been shown for CCR3 (Ponath *et al*.). In particular, the ability of RANTES to induce the directed migration ofmonocytes and a memory population of circulating T-cells (Schall, T. et al., Nature, 347:669-71 (1990)) suggests this chemokine and its receptor (s) may play 4 critical role in chronic inflammatory diseases, since these diseases are characterized by destructive infiltrates of T cells and monocytes.

Many existing drugs have been developed as antagonists of the receptors for biogenic amines, for example, as antagonists of the dopamine and histamine receptors. No successful antagonists have yet been developed to the receptors for the larger proteins such as chemokines and C5a. Small molecule antagonists of the interaction between C-C chemokine receptors and their ligands, including RANTES and MIP-1α, would provide compounds useful for inhibiting harmful inflammatory processes "triggered" by receptor ligand interaction, as well as valuable tools for the investigation of receptor-ligand interactions.

### SUMMARY OF THE INVENTION

It has now been found that a class of small organic molecules are antagonists of chemokine receptor function and can inhibit leukocyte activation and/or recruitment. An antagonist of chemokine receptorfunction is a molecule which can inhibit the binding and/or activation of one or more chemokines, including C-C chemokines such as RANTES, MIP-1α, MCP-2, MCP-3 and MCP-4 to one or more chemokine receptors on leukocytes and/or other cell types. As a consequence, processes and cellular responses mediated by chemokine receptors can be inhibited with these small organic molecules. Based on this discovery, a method of treating a disease associated with aberrant leukocyte recruitment and/or activation is disclosed as well as a method of treating a disease mediated by chemokine receptor function. The method comprises administering to a subject in need an effective amount of a compound or small organic molecule which is an antagonist of chemokine receptorfunction. Compounds or small organic molecules which have been identified as antagonists of chemokine receptor function are discussed in detail hereinbelow, and can be used for the manufacture of a medicament for treating or for preventing a disease associated with aberrant leukocyte recruitment and/or activation. In one aspect, the compound is represented by Structural Formula (I) 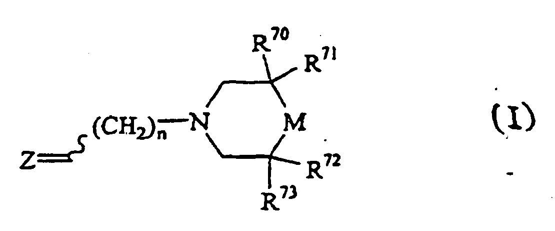 or a physiologically acceptable salt thereof, wherein Z, n, M, R⁷⁰, R⁷¹, R⁷² and R⁷³ are as described herein.

The invention also relates to the disclosed compounds and small organic molecules for use in treating or preventing a disease associated with aberrant leukocyte recruitment and/or activation. The invention also includes pharmaceutical compositions comprising one or more of the compounds or small organic molecules which have been identified herein as antagonists of chemokine function and a suitable pharmaceutical carrier.. The invention further relates to novel compounds which can be used to treat an individual with a disease associated with aberrant leukocyte recruitment and/or activation and methods for their preparation. The invention further relates to the compounds of the present invention for use in therapy.

### BRIEF DESCRIPTION OF THE DRAWINGS

Figure 1 is a schematic showing the preparation of the compounds represented by Structural Formula (I).
Figure 2 is a schematic showing the preparation of the compounds represented by Compound (VI-b).
Figure 3 is a schematic showing the preparation of the compounds represented by Structural Formula (I)
Figure 4 is a schematic showing the preparation of the compounds represented by Structural Formula (I), wherein Z is represented by Structural Formula (II) and wherein Ring A and/or Ring B in Z is substituted with R⁴⁰.
Figure 5 is a schematic showing the preparation of the compounds represented by Structural Formula (I), wherein Z is represented by Structural Formula (II) and wherein Ring A and/or Ring B in Z is substituted with -(O)ᵤ-(CH₂)ₜ-COOR²⁰, -(O)ᵤ-(CH₂)ₜ-OC(O)R²⁰, -(O)ᵤ-(CH₂)ₜ-C(O)-NR²¹R²² or -(O)ᵤ(CH₂)ₜ-NHC(O)O-R²⁰.
Figure 6 shows the preparation of compounds represented by Structural Formula (I), where in Z is represented by Structural Formula (II) and wherein Ring A or Ring B in Z is substituted with R⁴⁰.
Figure 7A is a schematic showing the preparation of 4-(4-chloraphenyl)-4-fluoropiperidine.
Figure 7B is a schematic showing the preparation of 4-4-azido-4-(4-chlorophenyl)piperidine.
Figure 7C is a schematic showing the preparation of 4-(4-chlorophenyl)-4-methylpiperidine.

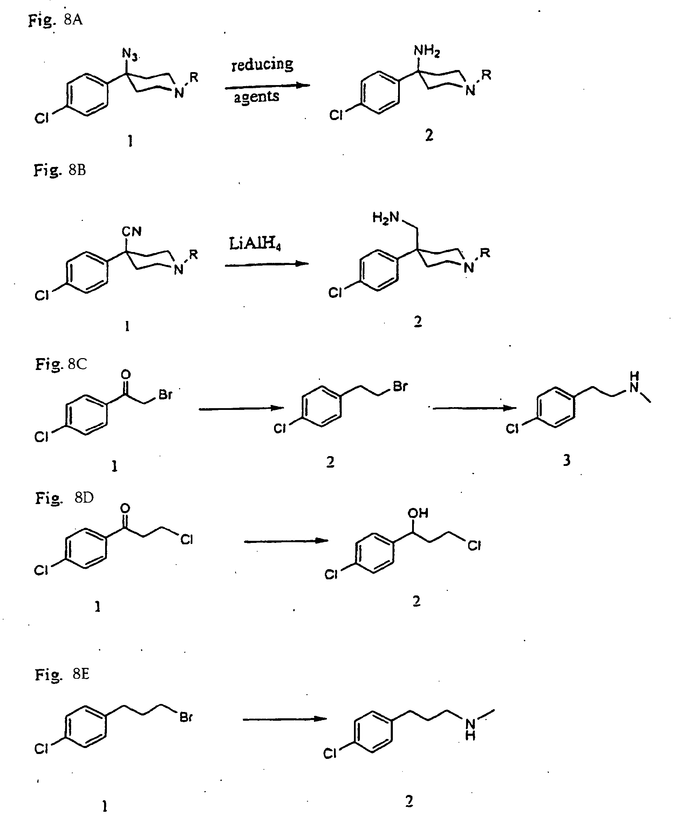

Figure 8A is a schematic showing the preparation of compounds represented by Structural Formula (I) wherein R¹ is an amine.
Figure 8B is a schematic showing the preparation of compounds represented by Structural Formulas (I) wherein R¹ is an alkylamine.
Figure 8C is a schematic showing the preparation of 2-(4-chlorophenyl)-1-(*N* methyl)ethylamine.
Figure 8D is a schematic showing the preparation of 3-(4-chlorophenyl)-3-chloro-1-hydroxypropane.
Figure 8E is a schematic showing the preparation of 3-(4-chlorophenyl)-1-*N*-methylaminopropane.
Figure 9A is a schematic showing the preparation of 3-(4-chlorophenyl)-3-hydroxyl-3-methyl-1-*N*-methylaminopropane.
Figure 9B is a schematic showing the preparation of 1 -(4-chlorobenzoyl)-1,3-propylenediamine.
Figure 9C is a schematic showing three procedures for the preparation of compounds represented by Structural Formula (I) wherein Z is represented by Structural Formula (II) and wherein Ring A or Ring B in Z is substituted with R⁴⁰. In Figure 9C, R⁴⁰ is represented by -(O)ᵤ-(CH₂)ₜC(O)-NR²¹R²², u is one, t is zero.
Figure 9D is a schematic showing the preparation of 4-(4-chlorophenyl)-4-pyridine.
Figure 10 is a schematic showing preparation of compounds of formula (VI c).
Figure 11 is a schematic showing preparation of compounds of formula (VI-e).
Figure 12 is a schematic showing a procedure for the preparation of Example 1.

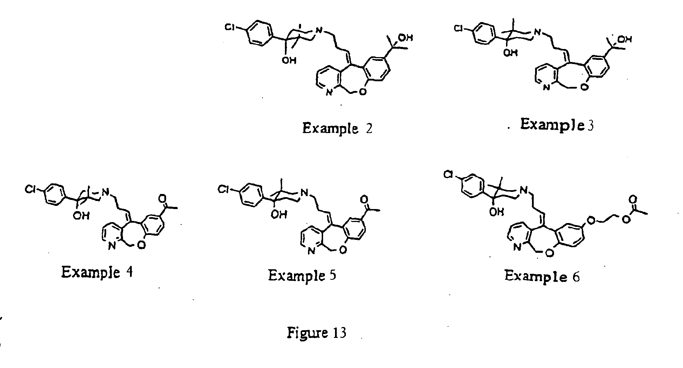

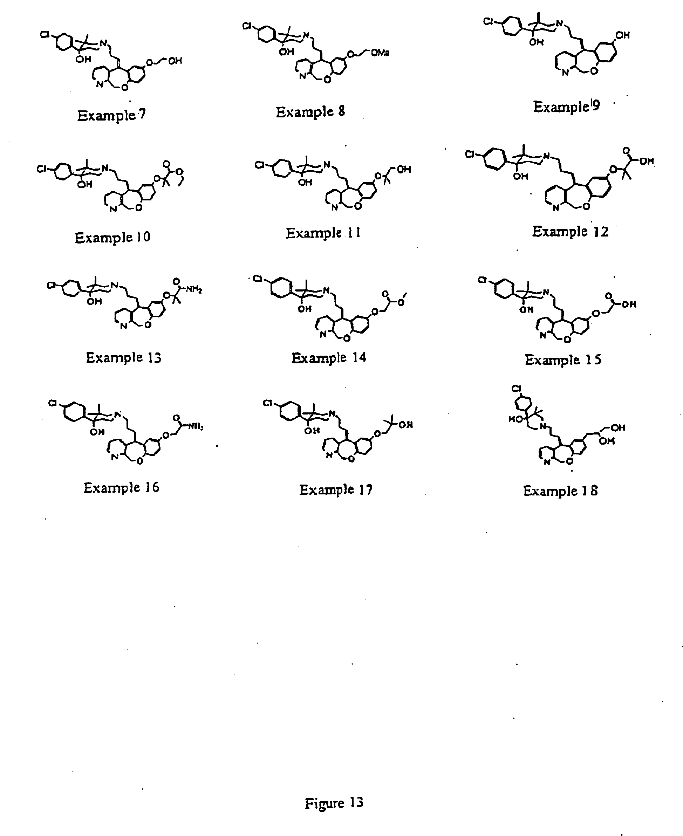

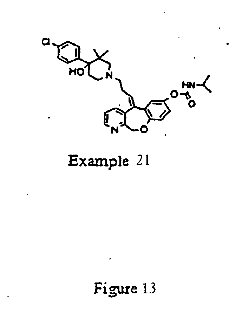

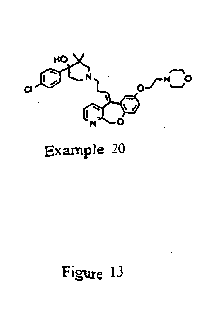

Figure 13 show the structures of exemplary compounds of the invention.

### DETAILED DESCRIPTION OF THE INVENTION

The present invention relates to small molecule compounds which are modulators of chemokine receptor function. In a preferred embodiment, the small molecule compounds are antagonists of chemokine receptor function. Accordingly, processes or cellular responses mediated by the binding of a chemokine to a receptor can be inhibited (reduced or prevented, in whole or in part), including leukocyte migration, integrin activation, transient increases in the concentration of intracellular free calcium [Ca⁺⁺]ᵢ, and/or granule release of proinflammatory mediators.

The invention further relates to the use of a compound of the present invention for the manufactured of a medicament for treating a disease associated with aberrant leukocyte recruitment and/or activation or mediated by chemokines or chemokine receptor function, including chronic inflammatory disorders characterized by the presence of RANTES. MIP-1 a, MCP-2, MCP-3 and/or MCP-4 responsive T cells, monocytes and/or eosinophils, including but not limited to diseases such as arthritis (e. g., rheumatoid arthritis), atherosclerosis, arteriosclerosis, restenosis, ischemia/reperfusion injury, diabetes mellitus (e.g., type 1 diabetes mellitus), psoriasis, multiple sclerosis, inflammatory bowel diseases such as ulcerative colitis and Crohn's disease, rejection of transplanted organs and tissues (i.e., acute allograft rejection, chronic allograft rejection), graft versus host disease, as well as allergies and asthma. Chronic inflammatory disease is a preferred condition. Multiple sclerosis is a further preferred condition. Arthritis, in particular rheumatoid arthritis, is also a preferred condition. Other diseases associated with aberrant leukocyte recruitment and/or activation which can be treated (including prophylactic treatments) with the methods disclosed herein are inflammatory diseases associated with Human Immunodeficiency Virus (HIV) infection, e.g., AIDS associated encephalitis, AIDS related mac-ulopapular skin eruption, AIDS related interstitial pneumonia, AIDS related enteropathy, AIDS related periportal hepatic inflammation and AIDS related glornerulo nephritis. The method comprises administering to the subject in need of treatment an effective amount of a compound (i.e., one or more compounds) which inhibits chemokine receptor function, inhibits the binding of a chemokine to leukocytes and/or other cell types, and/or which inhibits leukocyte migration to, and/or activation at, sites of inflammation.

The invention further relates to methods of antagonizing a chemokine receptor, such as CCR1, in a mammal comprising administering to the mammal a compound as described herein.

According to the method, chemokine-mediated chemotaxis and/or activation of pro-inflammatory cells bearing receptors for chemokines can be inhibited. As used herein, "pro-inflammatory cells" includes but is not limited to leukocytes, since chemokine receptors can be expressed on other cell types, such as neurons and epithelial cells.

While not wishing to be bound by any particular theory or mechanism, it is believed that compounds of the invention are antagonists of the chemokine receptor CCR1, and that therapeutic benefits derived from the method of the invention are the result of antagonism of CCR1 function. Thus, the method and compounds of the invention can be used to treat a medical condition involving cells which express CCR1 on their surface and which respond to signals transduced through CCR1, as well as the specific conditions recited above.

In the compounds of Structural Formula (I)
n is an integer, such as an integer from one to four. Preferably, n is one, two or three. More preferably n is two. In alternative embodiments, other aliphatic or aromatic spacer groups (L) can be employed for (CH₂)ₙ.
M is >NR² or >CR¹R². M is preferably >C(OH)R².
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴ or -NR³R⁴;
R¹ is preferably -H or -OH.
R² is -H, -OH, a halogen, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group), -O-(substituted or unsubstituted aliphatic group) or -C(O)-(substituted or unsubstituted aromatic group) or -C(O)-(substituted or unsubstituted aliphatic group). R² is preferably an aromatic group or a substituted aromatic group.
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group.
R¹ and R², R³ and R⁴, or R⁵ and R⁶ taken together with the atom to which they are bonded, can alternatively form a substituted or unsubstituted non-aromatic carbocyclic or heterocyclic ring.

In one embodiment, Z is a tricyclic ring system comprising two carbocyclic aromatic groups fused to a five, six, seven or eight membered cycloalkyl group or to a non-aromatic heterocyclic ring. In one example, Z is represented by Structural Formula (II):

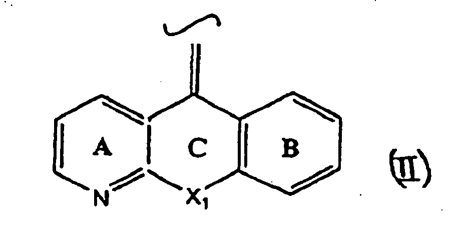

The rings in Structural Formula (II) labeled with an "A" and "B" are referred to herein as "Ring A" and "Ring B", respectively. The central ring, labeled with a "C"; is referred to as "Ring C" and can be, for example, a five, six, seven or eight membered non-aromatic carbocyclic ring (e.g., a cycloheptane or cyclooctane ring) or a non-aromatic heterocyclic ring. When Ring C is a non-aromatic heterocyclic ring, it can contain one or two heteroatoms such as nitrogen, sulfur or oxygen. When Z is represented by Structural Formula (II), the tricyclic ring system can be connected to the remainder of the molecule by a covalent double bond between a carbon atom in Ring C and the carbon atom which, as depicted in Structural Formula (I), is bonded to Z.

Ring A and/or Ring B in Structural Formula (II) can be unsubstituted. Alternatively, Ring A and/or Ring B can have one or more substituents. Suitable substituents are as described hereinbelow. In one example, Ring A or Ring B is substituted with -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² or -(O)ᵤ-(CH₂)₁-NHC (O)O-R²⁰_{.}
u is zero or one.
t is an integer, such as an integer from zero to three, and the methylene group - (CH₂)ₜ-can be substituted, as described herein for aliphatic groups, or unsubstituted.
R²⁰, R²¹ or R²² are independently-H, an aliphatic group, a substituted aliphatic group, an aromatic group, a substituted aromatic group or a non-aromatic heterocyclic group. Alternatively, R²¹ and R²², taken together with the nitrogen atom to which they are bonded, can form a non-aromatic heterocyclic ring.
X₁ is a bond, -O-, -S-, -CH₂-, -CH₂-CH₂-, -CH₂-S-, -5-CH₂-, -O-CH₂-, -CH₂-O-, -NRₜ-CH₂-, -CH₂-NR_{c}-, -SO-CH₂-, -CH₂-SO-, -S(O)₂-CH₂-, -CH₂-S(O)₂-, -CH=CH-, -NRₜ-CO- or -CO-NR_{c}-. Preferably X₁ is -CH₂-O-, -CH₂-CH₂-, -CH₂-S-, -NR_{c}-CO-or -CO-NR_{c}-.
R_{c} is hydrogen, an aliphatic group, a substituted aliphatic group, an aromatic group, a substituted aromatic group, a benzyl group or a substituted benzyl group.

In one example, R_{c} is -(CH₂)₃-COOR³⁰, -(CH₂)₅-C(O)-NR³¹R³² or -(CH₂)₅-NHC(O)-O-R³⁰, wherein s is an integer, such as an integer from one to three;

R³⁰, R³¹ and R³² are independently -H, an aliphatic group, a substituted aliphatic group, an aromatic group, a substituted aromatic group or a non-aromatic heterocyclic group. Alternatively, R³¹ and R³², taken together with the nitrogen atom to which they are bonded, form a non-aromatic heterocyclic ring.

In particularly preferred embodiments, Ring B in Structural Formula (II) is substituted para to the carbon atom of Ring B which is bonded to X₁ of Ring C, and Z is represented by Structural Formula (III)

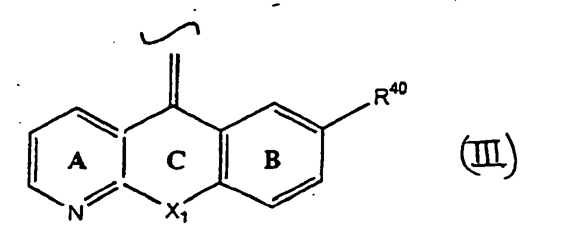

X₁ can be as described above in Structural Formula (II). Preferably X₁ is -CH₂-O-, -CH₂-CH₂- or -CH₂-S-. R⁴⁰ is -OH, -COOH, -NO₂, halogen, aliphatic goup, substituted aliphatic group, an aromatic group, a substituted aromatic group, -NR²⁴R²⁵, -CONR²⁴R²⁵, -NR²⁴C(O)-(aliphatic group), -NR²⁴C(O)-(substituted aliphatic group), -NR²⁴S (O)₂-(aliphatic goup), -NR²⁴S(O)₂-(substituted aliphatic group), -C(O)O-(aliphatic group), -C(O)O-(substituted aliphatic group), -C(O)-(aliphatic group), -C(O)-(substituted aliphatic group), -O-(aliphatic group), -O-(substituted aliphatic group), -O-(aromatic group), -O-(substituted aromatic group), an electron withdrawing group. -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰.
R²⁰, R²¹ or R²² are independently -H, an aliphatic group, a substituted aliphatic group, an aromatic group, a substituted aromatic group or a non-aromatic heterocyclic group t.
R²¹ and R²², taken together with the nitrogen atom to which they are bonded, form a non-aromatic heterocyclic ring. R²⁴ and R²⁵ are independently -H, an aliphatic group or a substituted aliphatic groups
U is zero or one.
t is an integer from zero to three.

Preferably R⁴⁰ is an aliphatic group, substituted aliphatic group, -O-(aliphatic group) or -O-(substituted aliphatic goup). In certain embodiments, R⁴⁰ is an -O-alkyl, such as -O-CH₃, -O-C₂H₅, -O-C₃H₇ or -O-C₄H₉.

In another embodiment, R⁴⁰ can be represented by -(O)ₙ-(CH₂)ₜ-C(O)-NR²¹R²², wherein u is one, t is zero, and R²¹ and R²² are as described herein. In this embodiment, R²¹ and R²² can each independently be -H, a substituted or unsubstituted aliphatic group, a substituted or unsubstituted aromatic group, or R²¹ and R²² taken together with the nitrogen atom to which they are bonded form a substituted or unsubstituted nonaromatic heterocyclic ring (e.g., pyrrolidine, piperidine, morpholine).

In another embodiment, R⁴⁰ can be represented by -(O)ᵤ-(CH₂)ₜ-C(O)-NR²¹R²², wherein u is zero, t is one to about three, and R²¹ and R²² are as described herein.

In another embodiment, R⁴⁰ can be represented by -(O)ᵤ-(CH₂)ₜ-C(O)-NR²¹R²², wherein both u and t are zero, and R²¹ and R²² are as described herein.

In another embodiment, R⁴⁰ is an aliphatic group (e.g., methyl, ethyl, propyl) that is substituted with -NR²⁴R²⁵or -CONR²⁴R²⁵, wherein R²⁴ and R²⁵ are as described herein. For example, R⁴⁰ can be represented by

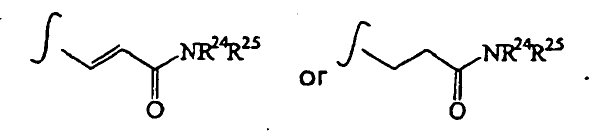

In a preferred embodiment, the chemokine receptor antagonist can be represented by Structural Formula I
wherein n is three, M is C(OH)R², R² is a phenyl group or a halophenyl group (e.g., 4-chlorophenyl) and Z is represented by Structural Formula (III) wherein X₁ is -CH₂-O-. In one example of this embodiment, R⁴⁰ can be -O-(substituted aliphatic group), such as

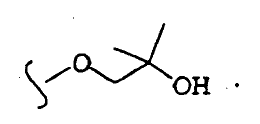

In particularly preferred embodiments, R⁴⁰ is

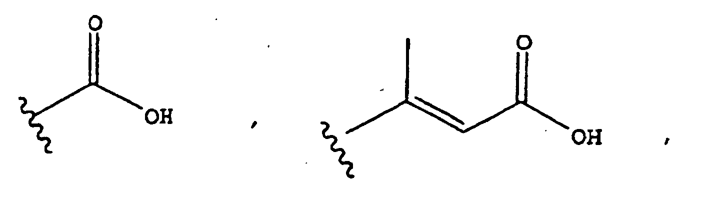

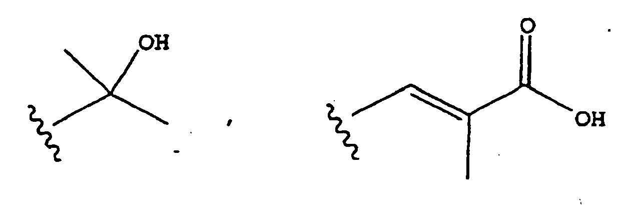

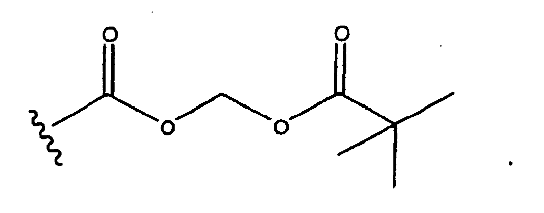

In other preferred embodiments, R⁴⁰ is a substituted aliphatic group, a substituted aromatic group, -O-substituted aliphatic group or -O-substituted aromatic group. Preferably the aliphatic or aromatic moiety of the substituted aliphatic group, substituted aromatic group, -O-substituted aliphatic group or -O-substituted aromatic group bears a substituent selected from the group consisting of-OH, -COOR -Q-aliphatic group or -Q-aromatic group substituent. Q is as described herein. Preferably, Q is -C(O)O-. For example, R⁴⁰ can be a linear, branched or cyclic aliphatic group that contains 1 to 6 carbon atoms, such as a C₁-C₆ alkyl group, a C₂-C₆ alkenyl, C₂-C₆ alkynyl, that is substituted with -OH, -COOH, -C (O)O-(C₁-C₆ aliphatic) or -C(O)O-(aromatic).

In a further embodiment, thechemotine receptor antagonist can be represented by structural formula (I) wherein Z is represented by structure Formula (III)
M is >CR¹R²,
R¹ is -H or -OH and
R² is a substituted aromatic group, wherein said substituted aromatic group is 4-halophenyl. In one example of this embodiment,
said 4-halophenyl is selected from the group consisting of 4-chlorophenyl, 4-bromophenyl and 4-fluorophenyl.

In a second example of this embodiment X₁ is -CH₂-O-.

In a third example ofthis embodiment, (at least one of R⁷⁰, R⁷¹, R⁷² and R⁷³ is an aliphatic group or a substititued aliphatic group; wherein
said aliphatic group is a C₁-C₆ alkyl and said substituted aliphatic group is a C₁-C₆ alkyl substituted with a substituent selected from the group consisting of -OH, -(O)ᵤ-(CH₂)ₜ-C(O)OR₂₀ and -O-(aliphatic group);
t is zero to three;
u is zero or one; and
R²⁰ is C₁-C₆ alkyl. In this third example, it is preferred that
R⁷⁰ and R⁷¹ are both -H; and
R⁷² and R⁷³ are independently selected from the group consisting of C₁-C₆ alkyl and substititued C₁-C₆ alkyl.

It is further preferred that R⁷² is -CH₃.

The ring containing M can have one or more suitable substituents which are the same or different Suitable substituents for the ring which contains M and other nonaromatic heterocyclic rings are as described herein. For example, the ring containing M can be substituted with a methyl, ethyl, propyl, butyl or oxo group.

R⁷⁰ and R⁷¹ are independently -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴, an acyl group, a substituted acyl group, a benzyl group, a substituted benzyl group, a non-aromatic heterocyclic group, a substituted non-aromatic heterocyclic group. -O-(substituted or unsubstituted aromatic group).

R⁷² and R⁷³ are independently -OH, -N₃, a halogen, an aliphatic group, a substituted aliphatic group, an aminoalkyl group, -O-(aliphatic group), -O-(substituted alipharic group), -SH, -S-(aliphatic group), -S-(substituted aliphatic group), -O-C(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴, an acyl group, a substituted acyl group, a benzyl group, a substituted benzyl group, a non-aromatic heterocyclic group, a substituted non-aromatic heterocyclic group. -O-(substituted or unsubstituted aromatic group);

In certain embodiments at least one of R⁷⁰, R⁷¹, R⁷² and R⁷³ is an aliphatic group or a substituted aliphatic group. Preferred aliphatic groups at R⁷⁰, R⁷¹, R⁷² and R⁷³ are C₁-C₆ alkyl, preferred substititued aliphatic groups are are C₁-C₆ alkyl substituted with -OH, -(O)ᵤ-(CH₂)ₜ-C(O)OR₂₀ or -O-(aliphatic group) wherein t is zero to three, u is zero or one, and R²⁰ is C₁-C₆ alkyl.

In a preferred embodiment, the chemokine receptor antagonist is represented by Structural Formula (1) wherein n is two; M is >C(OH)R²; R² is a halopbenyl group (*e*.*g*., 4-chlorophenyl); R⁷⁰ and R⁷¹ are -H and R⁷² and R⁷³ are independently C₁-C₆ alkyl or substituted C₁-C₆ alkyl; and
Z is represented by Structural Formula (III) wherein X, is -CH₂-O-

When R⁷² and R⁷³ are each -CH₃, the compounds of this preferred embodiment can have the formula: 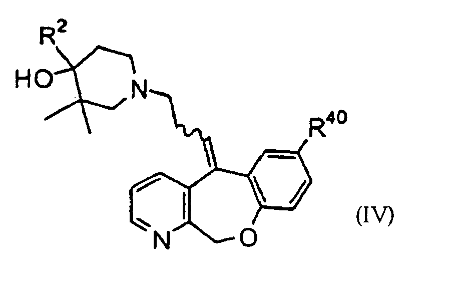 or a physiologically acceptable salt thereof wherein R² is 4-halophenyl Preferably R² is selected from the group consisting of 4-chlorophenyl, 4-bromophenyl and 4-fluorophenyl. Preferred groups at R⁴⁰ are as described herein. Particularly preferred at R⁴⁰ are aliphatic groups (*e*.*g*., C₁-C₆ alkyl) and substituted aliphatic groups.

In a particularly preferred embodiment, the compound is the (S)-enantiomer of the compound of Formula (v) and has the structure: 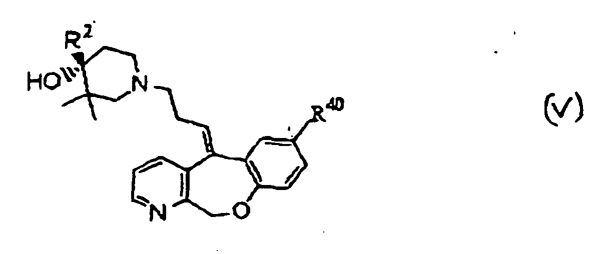 or a physiologically acceptable salt thereof, wherein
R² is 4-halophenyl; and
R⁴⁰ is selected from the group consisting of: 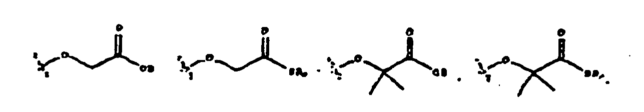 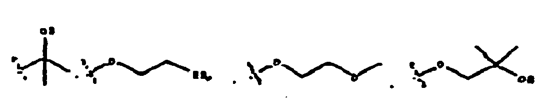 and

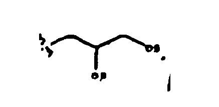

A preferred moiety for R⁴⁰ is

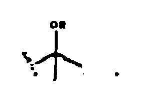

Preferred compounds of the invention one compounds of Formula (V) wherein R² is selected from the group consisting of 4-chlorophenyl, 4-bromophenyl and 4-fluorophenyl.

In one embodiment, the present invention provides a compound of Structured Formula (VI) 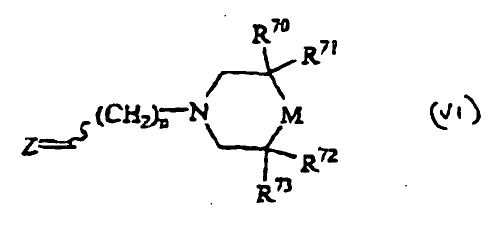 or physiologically acceptable salt thereof, wherein:
n is one to four;
M is >CR¹R²,
R¹ is -OH;
R² is 4-halophenyl;
R⁷⁰ and R⁷¹ are -H, and R⁷² and R⁷³ are -CH₃: or
R⁷⁰ and R⁷¹ are -CH₃, and R⁷³ and R⁷³ are -H;
Zis 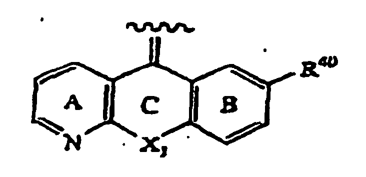 X₁ is -CH₂-O-; and
   R⁴⁰ is selected from the group consisting of: 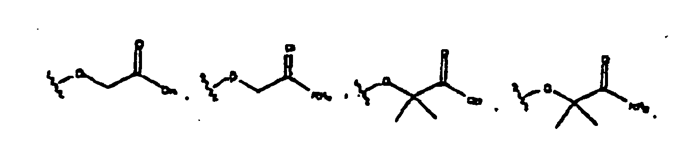 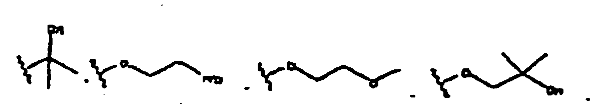 and

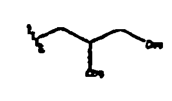

In the compounds of Structural Formula (VI), the 4-halophenyl is preferably selected from the group consisting of 4-chlorophenyl, 4-bromophenyl and 4-fluorophenyl.

In a preferred embodiment, ist the compounds of structural Formula (VI), R⁷⁰ and R⁷¹ are -H, R⁷² and R⁷³ are -CH₃, n is two, and the compound has the structure:

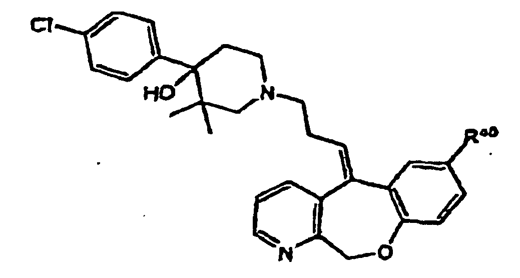

In a particularly preferred embodiment, R¹⁰ is

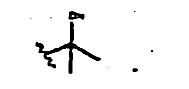

In particular embodiments, R⁵ is aliphatic group *(e*.*g*., C₁-C₆ alkyl) or substituted aliphatic group, and R⁶ is benzyl or substituted benzyl; or R⁵ and R⁶ taken together with the atom to which they are bonded, form a substituted or unsubstituted non-aromatic carbocyclic or heterocyclic ring. In more particular embodiments, R⁵ is C₁-C₆ alkyl and R⁶ is balo-substituted benzyl. In a preferred embodiment, R⁵ is ethyl and R⁶ is chloro-substituted benzyl (*e*.*g*., 4-chlorobenzyl).

The nitrogen atom in the ring containing M can be a tertiary nitrogen as depicted in Structural Formula (I), or the nitrogen atom can be quatemized with a suitable substituent, such as a C₁ to about C₆ or a C, to about C₃ substituted or unsubstituted aliphatic group. Compounds which comprise a quaternary nitrogen atom can also contain a counteranion such as chloride, bromide, iodide, acetate, perchlorate and the like.

The chemokine receptor antagonist described herein can be prepared and administered as active compounds or as prodrugs. Generally, prodrugs are analogues of pharmaceutical agents which can undergo chemical conversion by metabolic processes to become fully active. For example, A prodrug of the invention can be prepared by selecting appropriate groups for R⁴⁰. In one embodiment, a prodrug can be represented by Structural Formula (VII) 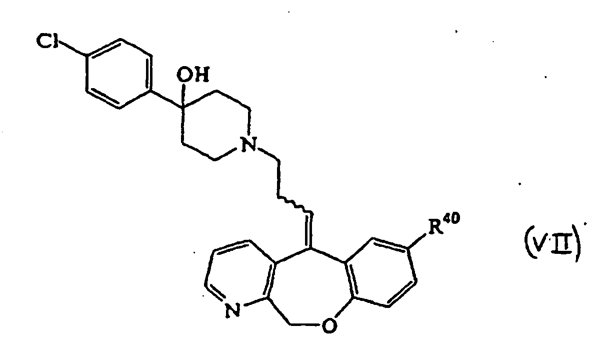 wherein, R⁴⁰ is Q-substituted aliphatic group, and the aliphatic group is substituted with -(O)ᵤ(CH₂)ₜC(O)OR²⁰, wherein Q is -C(O)O-, u is one, t is zero and R²⁰ is a cyclic aliphatic goup. For example, when the substituted aliphatic group is a substituted ethyl group, R⁴⁰ can be represented by:

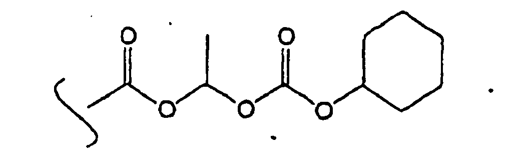

Such a prodrug can be converted to an active chemokine receptor antagonist represented by Structural Formula (VII) wherein R⁴⁰ is -COOH.

Another embodiment of the present invention includes novel compounds employed in these methods.

The compounds disclosed herein can be obtained as E- and Z-configurational isomers. It is expressly pointed out that the invention includes compounds of the E-configuration and the Z-configuration around the double bond, connecting Ring C of Z to the remainder of tbe molecule, and a method of treating a subject with compounds of the E-configuration, the Z-configuration, and mixtures thereof. Accordingly, in the structural formulas presented herein, the symbol: 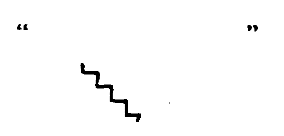 is used to represent both the E-configuration and the Z-configuration. Preferably Ring A and the alkylene chain bonded to Ring C are in the cis configuration. For example, the compounds can have the configuration of

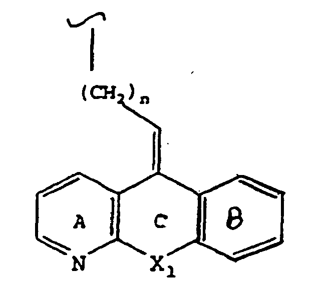

It is understood that one configuration can have greater activity than another. The desired configuration can be determined by screening for activity, employing the methods described herein.

Additionally, certain compounds of the invention may be obtained as different stereoisomers (e.g., diastereomers and enantiomers). The compounds of the invention can be prepared as racemates or as substantially pure stereoisomers. The stereoisomers of the invention (e.g., (*S*)- and (R)-enantiomers) can be prepared using any suitable method. For example, the enantiomers can be resolved from the racemate using chiral chromatography or recrystallization. Preferably, the stereoisomers (e.g., (*S*)- and/or (*R*)-enantiomers) are prepared by stereospecific synthesis as described herein.

The optical configuration of the stereoisomers of the invention are assigned using the (*R*),(*S*) method of Cahn-In-gold-Prelog. (See, J. March, "Advanced Organic Chemistry," 4th Edition, Wiley Interscience, New York, pp.109-111 (1992).)

The invention includes all isomeric forms and racemic mixtures of the disclosed compounds and a method of treating a subject with both pure isomers and mixtures thereof, including racemic mixtures. Sterioisomers can be separated and isolated using any suitable method, such as chromatography. Again, it is understood that one sterioisomer may be more active than another. The desired isomer determined by screening.

Also included in the present invention are physiologically acceptable salts of the compounds represented by Structural Formulas (I) through (VII) Salts of compounds containing an amine or other basic group can be obtained, for example, by reacting with a suitable organic or inorganic acid, such as hydrogen chloride, hydrogen bromide, acetic acid, citric acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium, ammonium, calcium and the like. (See, for example, Berge S.M. et al., "Pharmaceutical Salts," J. Pharma. Sci., 66:1 (1977).)

As used herein, aliphatic groups include straight chained, branched or cyclic C₁-C₂₀ hydrocarbons which are completely saturated or which contain one or more units of unsaturation. Preferred aliphatic groups are C₁ to about C₁₀ hydrocarbons. More preferred are C₁ to about C₆ or C, to about C₃ hydrocarbons. One or more carbon atoms in an aliphatic group can be replaced with a heteroatom, such as nitrogen, oxygen or sulfur. For example, suitable aliphatic groups include substituted or unsubstituted linear, branched or cyclic C₁-C₂₀ alkyl, alkenyl or alkynyl groups.

An aminoalkyl group is an alkyl group substituted with -NR²⁴R²⁵, R²⁴ and R²⁵ are as described herein. Preferably the alkyl moiety comprises one to about twelve, more preferably one to about six carbon atoms. The alkyl moiety of an aminoalkyl group can be unsubstituted or substituted as described herein for aliphatic groups. Examples of suitable aminoalkyl groups include aminomethyl, 2-aminoethyl, 3-aminopropyl, 4-aminobutyl, dimethylaminoethyl, diethylaminomethyl, methylaminohexyl, aminoethylenyl and the like.

Aromatic groups include carbocyclic aromatic groups such as phenyl, 1-naphthyl, 2-naphthyl, 1-anthracyl and 2-anthracyl, and heterocyclic aromatic or heteroaryl groups such as N-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl and 5-oxazolyl. Where these rings are fused, for example; to Ring C, the stated point of attachment can be either of the two fused bonds.

Aromatic groups also include fused polycyclic aromatic ring systems in which a carbocyclic aromatic ring or heteroaryl ring is fused to one or more other rings. Examples include tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 1-isoquinolinyl, 3-quinolinyl, 1-isoindolyl, 3-isoindolyl, acridinyl, 3-benzisoxazolyl, and the like. Also included within the scope of the term "aromatic group", as it is used herein, is a group in which one or more carbocyclic aromatic rings and/or heteroaryl rings are fused to a cycloalkyl or non-aromatic heterocyclic ring, for example, benzocyclopentane, benzocyclohexane.

Non-aromatic heterocyclic rings are non-aromatic carbocyclic rings which include one or more beteroatoms such as nitrogen, oxygen or sulfur in the ring. The ring can be five, six, seven or eight-membered and/orfused to another ring, such as a cycloalkyl on aromatic ring. Examples include 1,3-dioxolan-2-yl, 3-1 H-benzimidazol-2-one, 3-1-alkyl-benzimidazol-2-one, 3-1-methyl-benzimidazol-2-one, 2-tetrahydrofuranyl, 3-tetrahydrofuranyl, 2-tetrahyrothiophenyl, 3-tetrahyrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazalidinyl, diazolonyl, N-substituted diazolonyl, 1-phthalimidyl, 1-3-alkyl-phthalimidyl, benzoxane, benzopyrolidine, benzopiperidine, benzoxolane, benzothiolane, benzothiane, tetrahydrofuran-2-one-3-yl, 2,5-dihydro-5-oxo-4H-1,2,4-thiadiazot-3-yl, 2-oxo-3H-1,2,3,5-oxathiadiazol-4-yl,

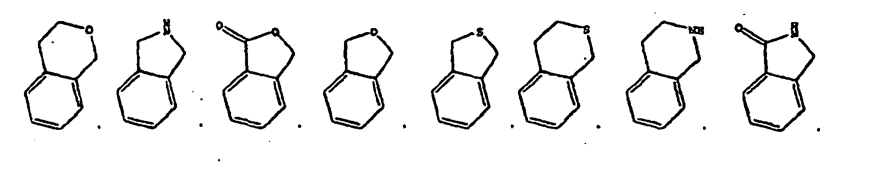

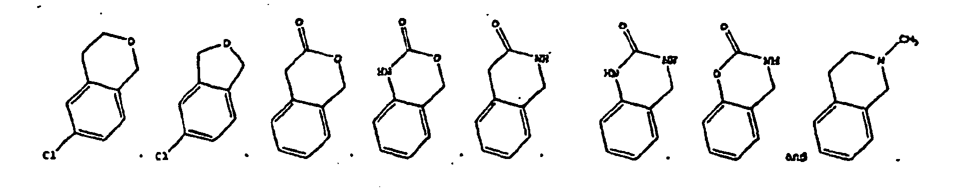

Suitable substituents on an aliphatic group, aromatic group (carbocyclic and heteroaryl), non-aromatic heterocyclic ring or benzyl group include, for example, an electron withdrawing group, a halogen (chloride, bromide, fluoride, iodide), azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, ureido, oxalo, amidino, -C(=NR⁶⁰)NR²¹R²²,=NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰,-Q-H, -Q-(aliphatic group),-Q-(substituted aliphatic group), -Q-(aryl), -Q-(aromadc group), -Q-(substituted aromatic group), -Q-(CH₂)ₚ-(substituted or unsubstituted aromatic group) p is an integer from 1-5), -Q-(non-aromatic heterocyclic group) or -Q-(CH₂)ₚ-(non-aromatic heterocyclic group).

R²⁰, R²¹ and R²² are independently -H, an aliphatic group, a substituted aliphatic group, an aromatic group, a substituted aromatic group, a non-aromatic heterocyclic group, -NHC(O)-O-(alipbatic group), -NHC(O)-O-(aromatic group) or-NHC(O)-O-(non-aromatic heterocyclic group) and wherein R²¹ and R²², taken together with the nitrogen atom to which they are bonded, can form a substituted or unsubstituted non-aromatic heterocyclic ring.

R⁶⁰ is a -H, -OH, -NH₂, an aromatic group or a substituted aromatic group t is an integer from zero to about three, and the methylene group, -(CH₂)ₜ-, can be substituted, as described herein for aliphatic groups, or unsubstituted.

u is zero or one.

Q is -O-,-S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-.

R²³ is -H, an aliphatic group, a benzyl group, an aryl group or non-aromatic heterocyclic group.

R²⁴ and R²⁵ are independently -H, -OH, an aliphatic group, a substituted aliphatic group, a benzyl group, an aryl group, non-aromatic heterocyclic group or R²⁴ and R²⁵ taken together with the nitrogen atom to which they are bonded can form a substituted or unsubstituted non-aromatic heterocyclic ring.

A substituted non-aromatic heterocyclic ring, benzyl group or aromatic group can also have an aromatic group, an aliphatic or substituted aliphatic group, as a substituent. When a non-aromatic ring (carbocyclic or heterocyclic) or an aromatic ring (carbocyclic aromatic or beteroaryl) is substituted with another ring, the two rings can be fused. A substituted aliphatic group can also have an oxo group, epoxy group, non-aromatic heterocyclic ring, benzyl group, substituted benzyl group, aromatic group or substituted aromatic group as a substituent. A substituted non-aromatic heterocyclic ring can also have =O, =S, =NH or =N(aliphatic, aromatic or substituted aromatic group) as a substituent. A substituted aliphatic, substituted aromatic, substituted non-aromatic heterocyclic ring or substituted benzyl group can have more than one substituent, which can be the same or different.

Acyl groups include substituted and unsubstituted aliphatic carbonyl, aromatic carbonyl, aliphatic sulfonyl and aromatic sulfonyl.

Suitable electron withdrawing groups include, for example, alkylimines, alkylsulfonyl, carboxamido, carboxylic alkyl esters, -CH=NH, -CN, -NO₂ and halogens. In the structural formulas depicted herein, the single or double bond by which a chemical group or moiety is connected to the remainder of the molecule or compound is indicated by the following symbol: 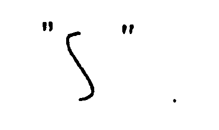 For example, the corresponding symbol in Structural Formulas (II) and (III) indicates the double bond by which the central ring of the tricyclic ring system is connected to the remainder of the molecule represented by Structural Formula (I).

A "subject" is preferably a bird or mammal, such as a human, but can also be an animal in need of veterinary treatment, e.g., domestic animals (e.g., dogs, cats, and the like), farm animals (e.g., cows, sheep, fowl, pigs, horses, and the like) and laboratory animals (e.g., rats, mice, guinea pigs, and the like).

An "effective amount" of a compound is an amount which results in the inhibition of one or more processes mediated by the binding of a chemokine to a receptor in a subject with a disease associated with aberrant leukocyte recruitment and/or activation. Examples of such processes include leukocyte migration, integrin activation, transient increases in the concentration of intracellular free calcium [Ca²⁺]ᵢ and granule release of proinflammatory mediators. Alternatively, an "effective amount" of a compound is a quantity sufficient to achieve a desired therapeutic and/or prophylactic effect, such as an amount which results in the prevention of or a decrease in the symptoms associated with a disease associated with aberrant leukocyte recruitment and/or activation.

The amount of compound administered to the individual will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compound can range from about 0.1 mg per day to about 100 mg per day for an adult. Preferably, the dosage ranges from about 1 mg per day to about 100 mg per day. An antagonist of chemokine receptor function can also be administered in combination with one or more additional therapeutic agents, e.g. theophylline, β-adrenergic bronchodilators, corticosteroids, antihistamines, antiallergic agents, immunosuppressive agents (e.g., cyclosporin A, FK-506, prednisone, methylprednisolone), hormones (*e*,*g*., adrenocorticotropic hormone (ACTH)), cytokines *(e*.*g*., interferons *(e*.*g*., IFNβ-1a, IFNβ-1b)) and the like.

The compound can be administered by any suitable route, including, for example, orally in capsules, suspensions or tablets or by parenteral administration. Parenteral administration can include, for example, systemic administration, such as by intramuscular, intravenous, subcutaneous, or intraperitoneal injection. The compound can also be administered orally (e.g., dietary), transdermally, topically, by inhalation (e.g., intrabronchial, intranasal, oral inhalation or intranasal drops), or rectally, depending on the disease or condition to be treated. Oral or parenteral administration are preferred modes of administration.

The compound can be administered to the individual in conjunction with an acceptable pharmaceutical or physiological carrier as part of a pharmaceutical composition for treatment of HIV infection, inflammatory disease, or the other diseases discussed above. Formulation of a compound to be administered will vary according to the route of administration selected (e.g., solution, emulsion, capsule). Suitable carriers may contain inert ingredients which do not interact with the compound. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% benzyl alcohol), phosphate-buffered saline, Hank's solution, Ringer's-lactate and the like. Methods for encapsulating compositions (such as in a coating of hard gelatin orcyclodextran) are known in the art (Baker, et al., "Controlled Release of Biological Active Agents", John Wiley and Sons, 1986).

The quantity of active ingredient (one or more compounds of the invention) in the composition can range from about 0.1 % to about 99.9% by weight. Preferably the quantity of active ingredient is about 10% to about 90%, or about 20% to about 80% by weight. A unit dose preparation can contain from 1 mg to about 1000 mg active ingredient, preferably about 10 mg to about 100 mg active ingredient. The composition can, if desired, also contain other compatible therapeutic agents, such as theophylline, β-adrenergic bronchodilators, corticosteroids, antihistamines, antiallergic agents, immunosuppressive agents (*e*.*g*., cyclosporin A, FK-506, prednisone, methylprednisolone), hormones (*e*.*g*., adrenocorticotropic hormone (ACTH)), cytokines (*e*.*g*., interferons *(e*.*g*., IFNβ-1a, IFNβ-1b)) and the like.

In one embodiment, the pharmaceutical composition comprises the (*S*)-enantiomer of a compound of the invention (*e*.*g*., a compound of Structural Formula (XIII)) and a physiologically acceptable carrier or excipient. For example, in one embodiment, the composition comprises (*S*)-4-(4-Chloro-phenyl)-1-{3-[7-(1-hydroxy-1-methyl-ethyl)-1 H-10-oxa-1-aza-dibenzo[a,d]cyclohepten-5-ylidene]-propyl}-3,3-dimethyl-piperidin-4-ol and a physiologically acceptable carrier or excipient. In certain embodiments, the pharmaceutical composition comprises the (*S*)-enantiomer of a compound of the invention (*e*.*g*., a compound of Structural Formula (XIII)) and is substantially free of the corresponding (*R*)-enantiomer (contains at least about 98% or at least about 99% enantiomeric excess of (*S*)-enantiomer). In another embodiments, the composition comprises the (S)-enantiomer of a compound of the invention (e.g., a compound of Structural Formula (XII)), the corresponding (R)-enantiomer and a physiologically acceptable carrier or excipient. In a more particular embodiment, the composition comprises a racemic compound of Structural Formula (XII), for example, racemic-4-(4-Chloro-phenyl)-1-{3-[7-(1-hydroxy-1-methyl-ethyl)-11H-10-oxa-1-aza-dibenzo[a,d]cyciohepten-5-ylidene]-propyl}-3,3-dimethyl-piperidin-4-ol In other embodiments, the ratio (*S*)-enantiomer: (*R*)-enantiomer (w/w) in the compositions is at least about 2:1 or about 5:1 or about 10:1 or about 20:1 or about 50:1.

The activity of compounds of the present invention can be assessed using suitable assays, such as receptor binding assays and chemotaxis assays. For example, as described in the Exemplification Section, small molecule antagonists of RANTES and MIP-1α binding have been identified utilizing THP-1 cells which bind RANTES and chemotax in response to RANTES and MIP-1α as a model for leukocyte chemotaxis. Specifically, a high through-put receptor binding assay, which monitors ²⁵I-R_{A}NTES and ¹²⁵I-MIP-1α binding to THP-1 cell membranes, was used to identify small molecule antagonists which block binding of RANTES and MIP-1α. Compounds of the present invention can also be identified by virtue of their ability to inhibit the activation steps triggered by binding of a chemokine to its receptor, such as chemotaxis, integrin activation and granule mediator release. They can also be identified by virtue of their ability to block RANTES and MIP-1α mediated HL-60, T-cell, peripheral blood mononuclear cell, and eosinophil chemotactic response.

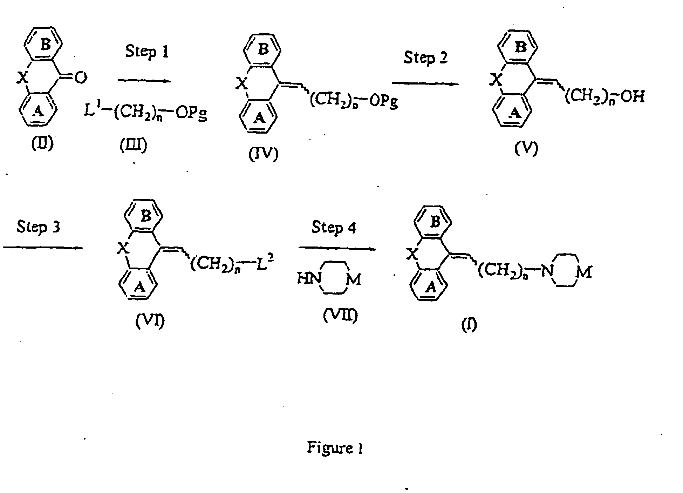

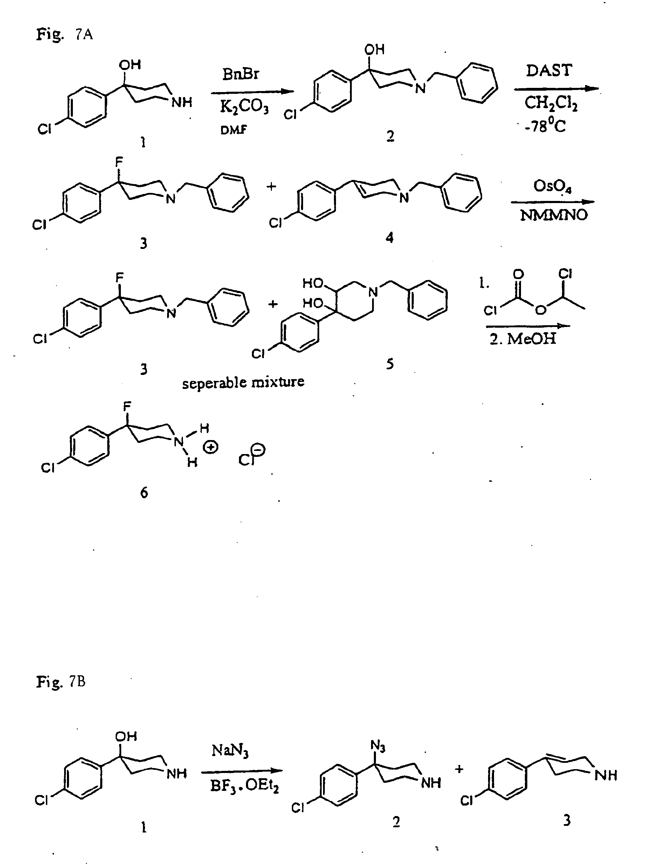

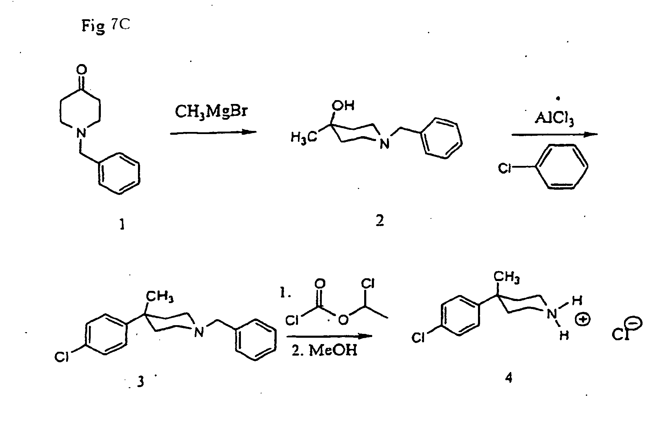

The compounds disclosed herein can be prepared accordingly to the schemes shown in Figures 1 - 5 and 7. The schemes are described in greater detail below.

Figure 1 shows the preparation of compounds represented by Structural Formula (I). L¹ is PPh₃Cl, PPh₃Br, PPh₃l or (EtO)₂P(O), L² is a suitable leaving group such as halogen, p-toluene sulfonate, mesylate, alkoxy, and phenoxy; Pg is a suitable protecting group such as tetrahydropyranyl; and the other symbols are as defined above.

In Step 1 of Figure 1, .a Wittig reaction is carried out in a solvent such as ether, ortetrahydrofuran (THF) in the presence of a base such as sodium hydride, n-butyl lithium or lithium diisopropylamide (LDA) at 0°C up to the reflux temperature for the solvent used for 5 minutes to 72 h. Compounds represented by Formula II in Figure 1 can be prepared by methods disclosed in JP 61/152673, U.S. Patent 5089496, WO 89/10369, WO 92/20681 and WO 93/02081, the entire teachings of which are incorporated herein by reference.

In Step 2 of Figure 1, deprotection is carried out with an acid in a solvent such as methanol at room temperature up to the reflux temperature for the solvent used for 5 minutes to 72 b. Alternatively, a compound of represented by Formula V in Figure 1 can be prepared directly from step 1 without isolating an intermediate. The reaction mixture obtained after the work up of the reaction described in step 1 can be dissolved in the solvent and reacted with the acid.

In Step 3 of Figure 1, the hydroxy group can be converted to a leaving group by known methods. Compounds represented by Formula VI in Figure 1 can be prepared by methods disclosed in J. Med. Chem., 1992 (35) 2074-2084 and JP 61/152673.

In Step 4 of Figure 1, an alkylation reaction is carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

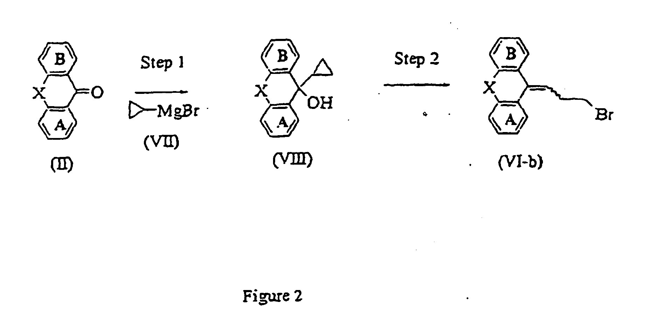

Figure 2 shows the preparation of compounds represented by Compound (VI-b). In Step 1 of Figure 2, a Grignard reaction may be carried outin a solvent such as ether, ortetrahydrofuran (THF) at 0°C up to the reflux temperature for the solvent used for 5 minuets to 72 h. Compound VII is available commercially.

In Step 2 of Figure 2, bromination may be carried out with brominate agents such as hydrobromic acid, bromotrimethylsilane or boron tribromide-methyl sulfide complex in a solvent such as acetic acid, dichloromethane or dichloroethane at room temperature up to the reflux temperature for the solvent used for 5 minutes to 72 h.

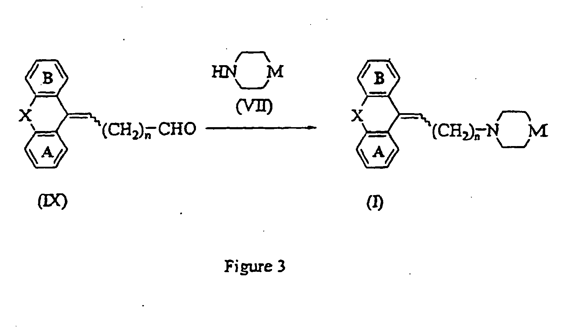

Figure 3 shows the preparation of compounds represented by Structural Formula (I). In Figure 3, a reductive amination may be carried out with reducing regents such as sodium cyanoborohydride, sodium acetoxyborohydride or sodium borohydride in a solvent such as methanol, ethanol, tetrahydrofuran (THF), dichloromethane or dichloroethane at room temperature up to the reflux temperature for the solvent used for 5 minutes to 72h.

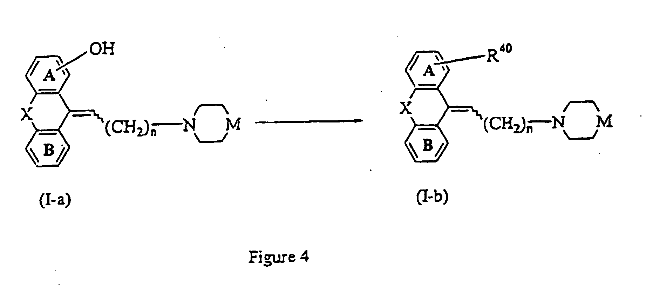

Figure 4 shows the preparation of compounds represented by Structural Formula (I), where in Z is represented by Structural Formulas (II) and wherein Ring A and/or Ring B in Z is substituted with R⁴⁰. In Figure 4, the alkylation reaction can be carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h..

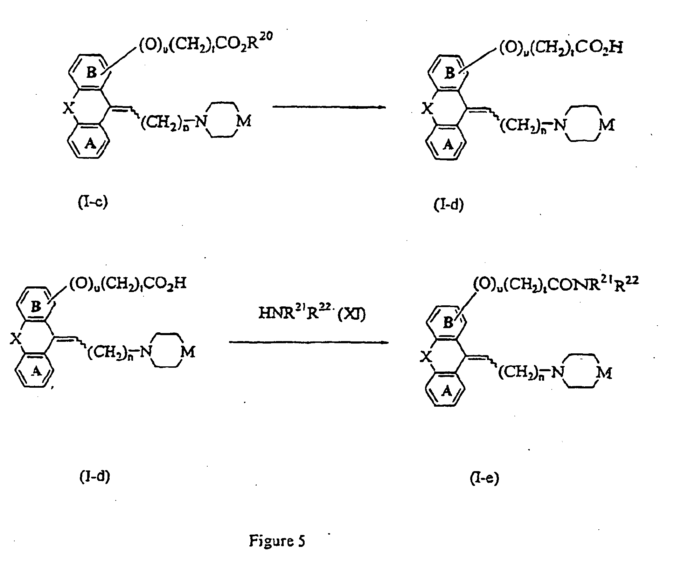

Figure 5 is a schematic showing the preparation of the compounds represented by Structural Formula (I),
wherein Z is represented by Structural Formulas (II) and wherein Ring A and/or Ring B in Z is substituted with -(O)ᵤ-(CH₂)ₜCOOR²⁰, -(O)ᵤ-(CH₂)ₜOC(O)R²⁰, -(O)ᵤ-(CH₂)ₜC(O)-NR²¹R²² Or -(O)ᵤ-(CH₂)ₜ-NHC(O)O-R²⁰. In Figure 5, the hydrolysis reaction may be carried out in a mixture of aqueous alkali metal hydroxide solution and a solvent such as methanol, ethanol, tetrahydrofuran (THF) or dioxane at room temperature up to the reflux temperature for the solvent used for 5 minutes to 72 h. The acylation reaction can be carried out using dicyclohexylcarbodiimide (DCC) or (1-ethyl-3-(3-dimethylaminopropyl)carbodiimide (DEC) in a solvent such as tetrahydrofuran (THF), dimethylformamide (DMF) or methylene chloride in the presence of a base such as pyridine or triethylamine (when necessary) at temperatures of 0 to 100°C for 5 minutes to 72 h.

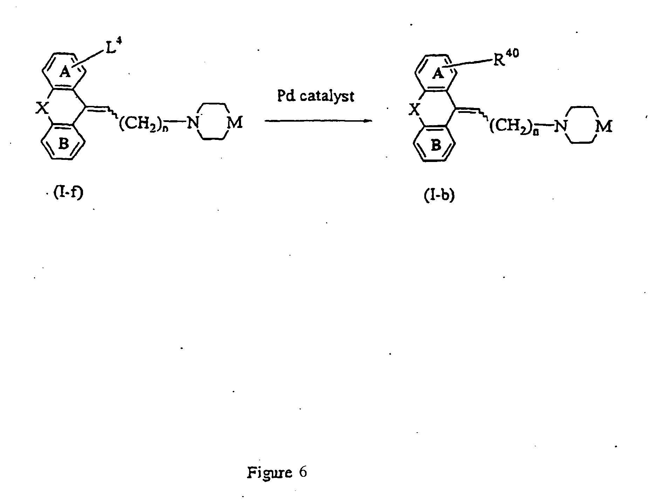

Figure 6 shows the preparation of compounds represented by Structural Formula (I), wherein Z is represented by Structural Formulas (II) and wherein Ring A or Ring B in Z is substituted with R⁴⁰. L⁴ is a suitable leaving group such as halogen or trifluoromethylsulfonate. In Figure 6, a palladium coupling reaction such as Stille coupling, Suzuki coupling, Heck reaction, or carboxylation using carbon monoxide may be carried out using a palladium catalyst such as tetrakis (triphenylphosphine)palladium, bis(triphenylphosphine)palladium chloride, and palladium acetate in a solvent such as tetrahydrofuran (THF), 1,4-dioxane, toluene, dimethylformamide (DMF), or dimethylsufoxide (DMSO) in the presence of additive (when necessary) such as triphenylphosphine, 1,1'-bis(diphenylphosphino)ferrocene, triethylamine, sodium bicarbonate, tetraethylammonium chloride, or lithium chloride at room temperature up to the reflux temperature for the solvent used for 5 minutes to 72 h.

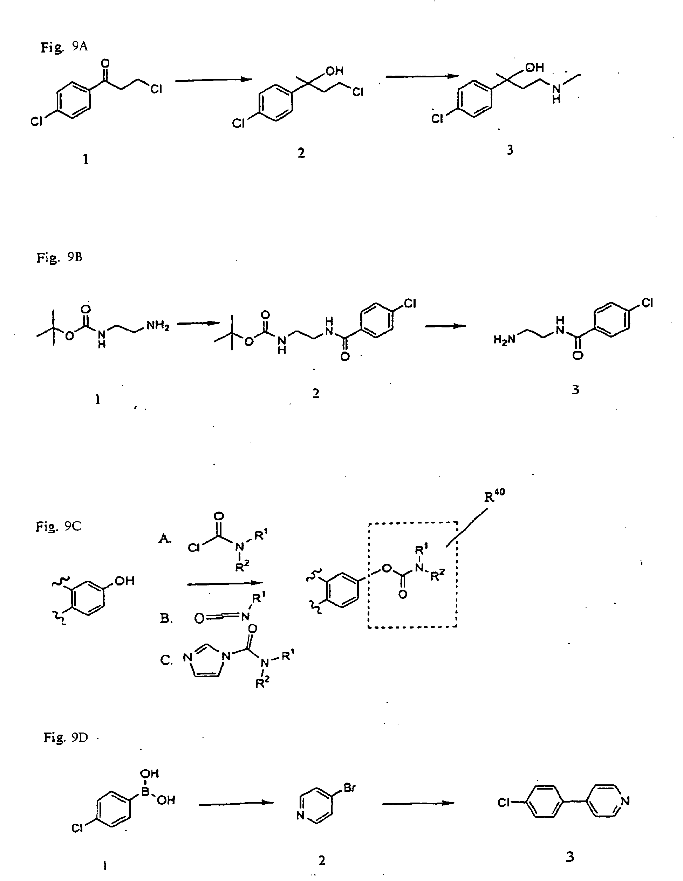

Figure 9C shows three procedures for the preparation of compounds represented by Structural Formulas (I),
wherein Z is represented by Structural Formula (II) and wherein Ring A or Ring B in Z is substituted with R⁴⁰ In Figure 9C, R⁴⁰ is represented by -(O)ᵤ-(CH₂)₁-C(O)-NR²¹R²², u is one, t is zero. In Figure 9C a compound containing a phenol can be reacted with a carbonate equivalent, such as a carbamoyl chloride (method A), an isocyanate (method B) or an acylimidazole (method C), in the presence of a base such as sodium hydroxide, potassium carbonate or sodium carbonate in a solvent such as dimethylformamide or tetrahydrofuran, at a temperature from 0°C to reflux temperature for a period of about 5 minutes to about 72 hours.

Compounds represented by Structural Formula (I), wherein Z is represented by Structural Formula (II), X is -CO-NR_{c}- and R_{c} is -(CH₂)₅-COOR³⁰, -(CH₂)₅C(O)-NR³¹R³² or -(CH₂)₅NHC(O)-O-R³⁰, can be prepared by suitable modification of the scheme shown in Figure 1 to 6. One modification utilizes the starting material shown in Figure 1, wherein X is -CO-NH-. The amide is then alkylated with L³-(CH₂)₅COOR³⁰, wherein L³ is a suitable leaving group, using the alkylation procedures described above. The remainder of the synthesis is as described In Figures 1 to 6.

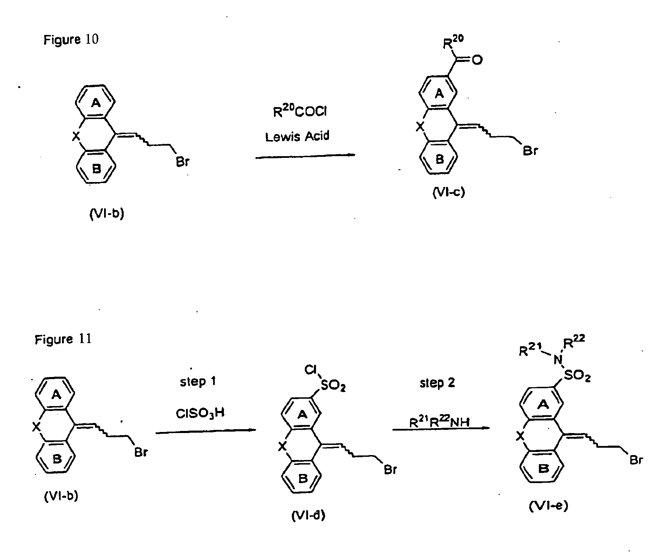

Figure 10 shows the preparation of compounds of formula a (VI-c). The Friedel-Crafts acylation can be carried out using an acid chloride in the presence of a Lewis acid, such as aluminum trichloride or titanium tetrachloride, in a solvent such as dichloromethane, dichloroethane, nitrobenzene or carbon disulfide. The acylation reaction can be run at a temperature of about room temperature up to the reflux temperature of the chosen solvent, and for a period of about 5 minutes to about 72 hours.

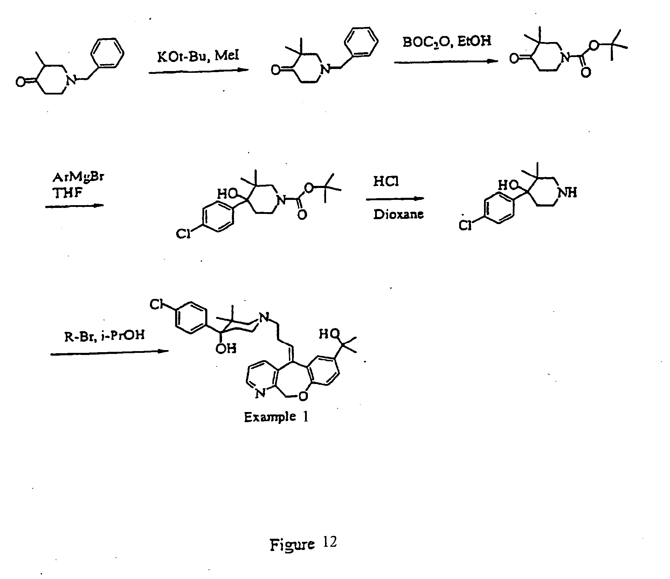

Figure 11 shows the preparation of compounds of formula (VI-e). In Step 1 of Figure 11, a chlorosulfonylation can be carried out using chlorosulfonic acid in a solvent, such as dichloromethane, or in the absence of a solvent at a temperature of about 0°C to about 60°C for a period of about 5 minutes to about 72 hours. In Step 2 of Figure 12, a coupling reaction can be carried out using an amine in the presence of a base, such as triethylamine, in a solvent such as dichloromethane, acetone, ethanol, THF or DMF. The reaction can be carried out at a temperature of about room temperature up to the reflux temperature of the selected solvent, and for a period of about 5 minutes to about 72 hours.

Although Figures 1 to 6, 10 and 11 show the preparation of compounds in which Rings A and B are phenyl rings, analogous compounds with heteroaryl groups for Rings A and B can be prepared by using starting materials with heteroaryl groups in the corresponding positions. These starting materials can be prepared according to methods disclosed in JP 61/152673, U.S. Patent 5089496, WO 89/10369, WO 92/20681 and WO 93/02081.

The invention is illustrated by the following examples which are not intended to be limiting in any way.

### EXEMPLIFICATION

### Membrane Preparations for Chemokine Binding and Binding Assays

Membranes were prepared from THP-1 cells (ATCC #TIB202). Cells were harvested by centrifugation, washed twice with PBS (phosphate-buffered saline). and the cell pellets were frozen at -70 to -85°C. The frozen pellet was thawed in ice-cold lysis buffer consisting of 5 mM HEPES (N-2-hydroxyethylpiperazine-N'-2-ethane-sulfonic acid) pH 7.5, 2 mM EDTA (ethylenediaminetetraacetic acid), 5 µg/ml each aprotinin, leupeptin, and chymostatin (protease inhibitors), and 100 µg/ml PMSF (phenyl methane sulfonyl fluoride - also a protease inhibitor), at a concentration of 1 to 5 x 10⁷ cells/ml. This procedure results in cell lysis. The suspension was mixed well to resuspend all of the frozen cell pellet. Nuclei and cell debris were removed by centrifugation of 400 x g for 10 minutes at 4°C. The supernatant was transferred to a fresh tube and the membrane fragments were collected by centrifugation at 25,000 x g for 30 minutes at 4°C. The supernatant was aspirated and the pellet was resuspended in freezing buffer consisting of 10 mM HERPES pH 7.5, 300 mM sucrose, 1 µg/ml each aprotinin, leupeptin, and chymostatin, and 10 µg/ml PMSF (approximately 0.1 ml per each 10⁸ cells). All clumps were resolved using a minihomogenizer, and the total protein concentration was determined using a protein assay kit (Bio-Rad, Hercules, CA, cat #500-0002). The membrane solution was then aliquoted and frozen at -70 to -85°C until needed.

Binding Assays utilized the membranes described above. Membrane protein (2 to 20 µg total membrane protein) was incubated with 0.1 to 0.2 nM ¹²⁵I-labeled RANTES or MIP-1α with or without unlabeled competitor (RANTES or MIP-1α) or various concentrations of compounds. The binding reactions were performed in 60 to 100 µl of a binding buffer consisting of 10 mM HEPES pH 7.2, 1 mM CaCl₂, 5 mM MgCl₂, and 0.5% BSA (bovine serum albumin), for 60 min at room temperature. The binding reactions were terminated by harvesting the membranes by rapid filtration through glass fiber filters (GF/B or GF/C, Packard) which were presoaked in 0.3% polyethyleneimine. The filters were rinsed with approximately 600 µl of binding buffer containing 0.5 M NaCl, dried, and the amount of bound radi oactivity was determined by scintillation counting in a Topcount beta-plate counter.

The activities of test compounds are reported in the Table below as IC₅₀ values or the inhibitor concentration required for 50% inhibition of specific binding in receptor binding assays using ¹²⁵I-RANTES or ¹²⁵I-MIP-1α as ligand and THP-1 cell membranes. Specific binding is defined as the total binding minus the non-specific binding; non-specific binding is the amount of cpm still detected in the presence of excess unlabeled Rantes or MIP-1α.

**Table BIOLOGICAL DATA**

| Example | IC50 (µM) |
|---|---|
| 2 | <1 |
| 3 | <1 |
| 4 | <1 |
| 5 | <1 |
| 6 | <1 |
| 7 | <1 |
| 8 | <1 |
| 9 | <1 |
| 10 | <1 |
| 11 | <1 |
| 12 | <1 |
| 13 | <1 |
| 14 | <1 |
| 15 | <1 |
| 16 | <1 |
| 17 | <1 |
| 18 | <1 |
| 19 | <1 |
| 20 | <1 |
| 21 | <1 |

### Example

### 4-(4-Chloro-phenyl)-1- {3-[7-(1-hydroxy-1-methyl-ethyl)-11H-10-oxa-1-azadibenzo[a,d]cyclohepten-5-ylidene]-propyl}-3,3-dimethyl-piperidin-4-ol

### Step 1: 1-Benzyl-3,3-dimethyl-piperidin-4-one

To a solution of 1-Benzyl-3-methyl-piperidin-4-one (2.03 g, 10 mmol) in THF (10 mL) was added potassium t-Butoxide (1.1 g, 10 mmol) and methyl iodide ( 0.62 mL, 10 mmol). The solution was then stirred at room temperature for about 72 hours. The reaction was quenched with brine and extracted with ethyl acetate. The combined organic layers were concentrated in *vacuo,* then purified by flash chromatography on silica gel (35g SiO₂, gradient elution from 100% hexane to 100% ethyl acetate) to yield the title compound as a colorless oil. ¹H-NMR (CDCL₃) ∂:1.12 (6H, s), 2.41 (2H, s), 2.52 (2H, m), 2.73 (2H, m), 3.56 (2H, s), 7.20-7.40 (5H, m). ESI-MS m/z: 218(M+1).

### Step 2: 3,3-Dimethyl-4-oxo-piperidine-1-carboxylic acid tert-butyl ester

To a solution of 1-Benzyl-3,3-dimethyl-piperidin-4-one (2.48 g, 11 mmol) in ethanol (100 mL) was added di-tert-butyl dicarbonate (2.18, 10 mmol) and palladium hydroxide (0.10 g), The suspension was then shaken under a hydrogen atmosphere (40 PSI) at room temperature for about an additional 12 hours. The reaction was filtered through celite and evaporated *in vacuo* to yield the title compound as a white solid.
¹H-NMR (CDCL₃) ∂: 1.12 (6H, br s), 1.48 (9H, s), 2.45-2.80 (3H, m), 3.12 (1 H, m), 3.42 (1 H, br s), 3.70 (1 H, m).

### Step 3: 4-(4-Chloro-phenyl)-4-hydroxy-3,3-dimethyl-piperidine-1-carboxylic acid tert-butyl ester

To an ice-cooled solution 3,3-Dimethyl-4-oxo-piperidine-1-carboxylic acid tert-butyl ester (1.6 g, 7.2 mmol) in THF (20 mL) was added 4-chlorophenylmagnesium bromide (1 M in ether, 15 mL, 15 mmol). The solution was allowed to warm to about room temperature, then stirred at room temperature for about 22 hours. The reaction was quenched with aqueous ammonium chloride and extracted with ethyl acetate. The combined organic layers were concentrated in *vacuo*, then purified by flash chromatography on silica gel (35g SiO₂, gradient elution from 100% hexane to 100% ethyl acetate) to yield the title compound as a colorless oil.
¹H-NMR (CDCL₃) ∂: 1.12 (6H, s), 1.50 (11 H, m), 2.60 (1 H, m), 3.20 (2H, m), 3.56 (1 H, m), 4.20 (1 H, m), 7.20-7.40 (4H, m). ESI-MS m/z: 218 (M + 1).

### Step 4: 4-(4-Chloro-phenyl)-3,3-dimethyl-piperidin-4-ol

4-(4-Chloro-phenyl)-4-hydroxy-3,3-dimethyl-piperidine-1-carboxylic acid tert-butyl ester (0.25 g, 0.73 mmol) was dissolved in 4M HCl/Dioxane (2 mL, 8 mmol). The solution was stirred at room temperature for about 4 hours. The solvent was removed in *vacuo.* The residue was quenched with aqueous sodium hydroxide, extracted with ethyl acetate, and the organic layers were dried over sodium sulfate and evaporated *in vacuo* to yield the title compound as a yellow solid. The mixture was carried on to the next step vithout further purification.
4-(4-Chloro-phenyl)-1- {3-[7-(1-hydroxy-1-methyl-ethyl)-11H-10-oxa-1-azadibenzo[a,d]cyclohepten-5-ylidene]-propyl}-3,3-dimethyl-piperidin-4-ol

To a solution of the amino alcohol mixture (0.17 g, 0.7mmol) in isopropanol (5mL) was added 2,6-lutidine (0.23 mL, 2.0 mmol) and catalytic potassium iodide. This mixture was heated to about 80°C, and treated with 2-[5-(3-Bromo-propylidene)-5,11-dihydro-10-oxa-1-aza-dibenzo[a,d]cyclohepten-7-yl]-propan-2-ol (0.19 g, 0.5 mmol), added in portions over about 2 hours. The solution was then stirred at about 80°C for an additional 2 hours. The reaction was concentrated *in vacuo,* then purified by flash chromatography on silica gel (10g SiO₂, gradient elution from 100% ethyl acetale to 87% ethyl acetate: 10% methanol:3% triethylamine) to yield the title compound as a brown semisolid.
¹H-NMR (CDCL₃) ∂: 0.78 (3H, s), 0.90 (3H, s), 1.45 (2H, m), 1.53 (6H, s), 2.28-2.80 (9H, m), 5.30 (2H, brs), 6.15 (1H, t, J = 1.4 Hz ), 6.79 (2H, d, J = 8.4 Hz), 7.18-7.45 (7H, m), 7.59 (1 H, d, J = 8 Hz), 8.45 (1 H, m). ESI-MS m/z: 533 (M + 1).

### Example 2

### S-4-(4-Chloro-phenyl)-1- {3-[7-(1-hydroxy-1-methyl-ethyl)-11 H-10-oxa-1-azadibenzo[a,d]cyclohepten-5-ylidene]-propyl}-3,3-dimethyl-piperidin-4-ol

### Step 1

To a dry, 2L 2-neck, round-bottom flask equipped with a magnetic stirrer, a condenser, and a large 10°C water bath was added 4-oxo-piperidine-1-carboxylic acid tert-butyl ester (125 g, 628 mmol) and anhydrous tetrahydrofuran (1 L). To the resulting yellow solution was added methyl iodide (85 mL, 1365 mmol). Sodium t-butoxide (150g, 1560 mmol) was then added portionwise over 30 minutes. An exotherm was detected, especially at the beginning of the addition. The reaction mixture did warm to a gentle reflux, the rate was controlled by the speed of addition of base. The mixture was stirred an additional 30 minutes. The solvent was removed *in vacuo.* The oily residue was treated with NH₄Cl/water (500 mL), and extracted with ether (3 x 200 mL). The combined organics were washed with brine, dried over Na₂SO₄, and filtered through a short plug of silica gel. The solvent was removed in *vacuo,* and the resulting yellow oil had started to ccrystallize. It was left under high vacuum overnight. The mixture was slurried in hexane (50-100 mL) and sonicated for one minute. The yellow solid was collected by filtration and washed with hexane (100 mL). The first crop of 3,3-dimethyl-4-oxo-piperidine-1-carboxylic acid tert-butyl ester yielded a yellow solid. (See, preparation of (37) in Vice, S. et al., J. Org. Chem., 66:2487-2492 (2001))
¹H-NMR (CDCl₃, 300 MHz) δ: 1.13 (s, 6 H), 1.49 (s, 9 H), 2.49 (t, 2 H), 3.43 (br s, 2H), 3.73 (t, 2 H).

### Step 2

A 2-neck, 2-L round bottom flask was fitted with 2 125 mL dropping funnels and a stir bar. The assembly was flame-dried under dry nitrogen. The flask was charged with THF (700 mL) and 4-bromo-chlorobenzene (33.7 g, 176 mmol, 2.5 eq.). The resulting solution was cooled to -78 °C in a dry ice/acetone bath. To one of the dropping funnels was added butyllithium (2.5 M in hexanes, 70 mL, 175 mmol, 2.5 eq) via canula. The butyllithium solution was slowly added to the cold THF solution over 1 h. Stirring continued for an additional 0.5 h affording a white suspension. A solution of 3,3-dimethyl-4-oxo-piperidine-1-carboxylic acid tert-butyl ester (16.0 g, 70.5 mmol,1 eq.) in THF (100 mL) was prepared and added to the reaction mixture via the second dropping funnel over 1.75 h. The resulting mixture was stirred at -78 °C for 2h, at which time reaction appears to be essentially complete by TLC analysis. Saturated aqueous NH₄Cl (150 mL) was added and the reaction was allowed to warm to rt. Water (150 mL) was added and the mixture extracted with ethyl acetate (2 + 1 L). The combined extracts were washed with water and brine, dried over magnesium sulfate, filtered and concentrated. The solid residue was triturated with ethyl acetate and filtered. The supernatant was concentrated and triturated with ether. The resulting supernatant was then triturated - with ether/petroleum ether. The resulting solids were combined to afford 4-(4-Chloro-phenyl)-4-hydroxy-3,3-dimethyl-piperidine-1-carboxylic acid tert-butyl ester (17.52 g, 51.6 mmol, 73 %) as an off-white solid.
¹H-NMR (CDCl₃, 300 MHz) δ: 0.82 (s, 6 H), 1.34-1.44 (m, 2 H), 1.49 (s, 9 H). 2.67 (ddd, 1 H), 3.10 - 3.70 (m, 3 H), 4.00 - 4.30 (m, 1 H), 7.31 (d, 2 H), 7.39 (d, 2 H).

### Step 3

To a cooled (0 °C) solution of the compound prepared in step 2 (10.42 g, 30.7 mmol) in methylene chloride (300 mL) was slowly added trifluoroacetic acid (60 mL) over 1.25 h. The resulting yellow solution was stirred at 0 °C for an additional 1.5 h. The mixture was concentrated under reduced pressure and the residue dissolved in ethyl acetate (1.2 L), washed with aqueous sodium hydroxide (1N, 150 mL). The aqueous layer was extracted with additional ethyl acetate (200 mL) and the combined extracts were washed with brine, dried over sodium sulfate, filtered and concentrated. The resulting solid residue was triturated with etherto afford 4-(4-chloro-phenyl)-3,3-dimethyl-piperidin-4-ol (6.94 g, 29.0 mmol, 94 %) as an off white solid.
¹H-NMR (CD₃OD, 300 MHz) δ: 0.73 (s, 3 H), 0.85 (s, 3 H), 1.42 (ddd, 1 H), 2.36 (d, 1 H), 2.61 (ddd, 1 H), 2.91 (br dd, 1 H), 3.08 - 3.19 (m, 2 H), 7.26 - 7.32 (m, 2 H), 7.44-7.50 (m, 2 H).
MS m/z: 240 (M + 1).

### Step 4

A visibly clean 5 L, 3-neck flask was fitted with an overhead stirrer and flushed with nitrogen for 20 min. 4-(4-Chloro-phenyl)-3,3-dimethyl-pipendin-4-ol (202g, 843 mmol), L-(+)-tartaric acid (114 g, 759 mmol) and 4040 mL of a9:1 butanone:water mixture were added to the flask. The mixture was heated to reflux. Water (202 mL) was added portionwise over 45 min (ratio of butanone to water: 6:1) to fully dissolve the solid mixture. Reflux was continued an additional 45 min, the heat source was then turned off and the flask allowed to cool slowly to rt - overnight. Solids were removed under suction filtration and dried 3 d in *vacuo* to afford S-enantiomer (134.4 g, 41 %) as the L-(+) tartrate salt. The above salt was partitioned between 1 M NaOH and methylene chloride (brine washed and sodium sulfate-dried) to afford the free base.
¹H-NMR (CD₃OD, 300 MHz) δ: 0.73 (s, 3 H), 0.85 (s, 3 H), 1.42 (ddd, 1 H), 2.36 (d, 1 H), 2.61 (ddd, 1 H), 2.91 (br dd, 1 H), 3.08 - 3.19 (m, 2 H), 7.26 - 7.32 (m, 2 H), 7.44 - 7.50 (m, 2 H).
MS m/z: 240 (M + 1).

### Step 5

To a solution of the homochiral product of step 4 (2.40 g, 10 mmol) in acetonitrile (80 mL) and water (20 mL) was added potassium carbonate (1.39 g, 10 mmol) followed by 2-[5-(3-bromo-propylidene)-5,11-dihydro-10-oxa-1-aza-dibenzo[a,d]cyclohepten-7-yl]-propan-2-ol (2.49 g, 6.67 mmol). The bipbasic mixture was stirred at rt 48 h. Acetonitrile was removed by rotary evaporation and the resulting slurry was partitioned between ethyl acetate and brine. The organic phase was dried over magnesium sulfate, filtered and concentrated to afford an oily solid which was purified by silica gel chromatography (methylene chloride - 90/10 methylene chloride/methanol gradient) to afford the title compound as an off-white powder (2.63 g, 74 %).
¹H-NMR (CD₃OD, 300 MHz) δ: 0.71 (s, 3 H), 0.84 (s, 3 H), 1.4-1.55 (m, 9 H), 2.18- 2.81 (m, 9 H), 5.15 - 5.40 (br s, 2 H), 6.23 (t, 1 H), 6.74 (d, 1 H), 7.23 - 7.31 (m, 3 H), 7.42 - 7.50 (m, 4 H), 7.80 (dd, 1 H), 8.47 (dd, 1 H).
MS m/z: 533 (M + 1).

### Example 3

### R-4-(4-Chloro-phenyl)-1- (3-[7-(1-hydroxy-1-methyl-ethyl)-11 H-10-oxa-1-azadibenzo[a,d]cyclohepten-5-ylidene]-propyl)-3,3-dimethyl-piperidin-4-ol

### Step 1

Racemic 4-(4-chloro-phenyl)-3,3-dimethyl-piperidin-4-ol (0.500 g, 2.086 mmol) was dissolved in minimal hot isopropyl alcohol (ca. 5 mL). The hot solution was filtered through a plug of cotton and transferred to a solution of (1 S) -(+)-10-camphorsulfonic acid (0,484 g, 2.086 mmol) in isopropyl alcohol (ca. 3 mL). The mixture was stirred vigorously for several minutes, during which a thick precipitate forms, and allowed to cool to rt over 0.25 h. The solids were removed by suction filtration and dried *in vacuo.* The dried salt was dissolved in hot isopropyl alcohol (ca. 50 mL), filtered thorugh a cotton plug, and allowed to slowly cool to rt, undisturbed, overnight. The solids that formed on cooling (95 mg, 19 % of theoretical) were removed by suction filtration and shown by analytical HPLC to be enantiomerically pure. The salt was suspended in ethyl acetate and neutralized with sodium hydroxide (1 N). The homogenous organic phase was washed with water and brine, dried oversodium sulfate, filtered and dried to afford *R*-4-(4-chlorophenyl)-3,3-dimethyl-piperidin-4-ol.
¹H-NMR (CD₃OD, 300 MHz) δ: 0.73 (s, 3 H), 0.85 (s, 3 H), 1.42 (ddd, 1 H), 2.36 (d, 1 H), 2.61 (ddd, 1 H), 2.91 (br dd, 1 H), 3.08 - 3.19 (m, 2 H), 7.26 - 7.32 (m, 2 H), 7.44 - 7.50 (m, 2 H).
MSm/z:240(M+1).

### Step 2

The titled compound was prepared following the procedure in step 5 of Example 2, substituting S-4-(4-chloro-phenyl)-3,3-dimethyl-piperidin-4-ol for R-4-(4-chloro-phenyl)-3,3-dimethyl-piperidin-4-ol.
¹H-NMR (CD₃OD, 300 MHz) δ; 0:1 (s, 3 H), 0.84 (s, 3-H), 1.4-1.55 (m, 9 H), 2.18- 2.81 (m, 9 H), 5.15- 5.40 (br s,2 H), 6.23 (t, 1H), 6.74 (d,1 H), 7.23- 7.31 (m, 3 H), 7.42- 7.50 (m, 4 H), 7.80 (dd, 1 H), 8.47 (dd, 1 H).
MS m/z: 533 (M + 1).

### Example 4

### S-1-(5- {3-[4-(4-Chloro-phenyl)-4-hydroxy-3,3-dimelhyl-piperdin-1-yl]-propylidene}-5,11-dihydro-10-oxa-1-aza-dibenzo [a,d]cyclohepten-7-yl)-ethanone

To a solution of S-4-(4-chloro-phenyl)-3,3-dimethyl-piperdin-4-ol (180 mg, 0.75 mmol) in acetonitrile/water (4/1) was added potassium carbonate (120 mg, 0.86 mmol), followed by 1-[5-(3-bromo-propylidene)-5,11-dihydro-10-oxa-1-azadibenzo[a,*d*]cyclohepten-7-yl]-ethanone (214 mg, 0.6 mmol). The reaction mixture was stirred at 50 °C for 8 hours and then concentrated *in vacuo.* The resulting residue was treated with water and extracted with ethyl acetate. Solvent was evaporated from the combined dried (MgSO₄) organic extracts, and the residue was purified by column chromatography on silica gel using methylene chloride-methanol (96:4) to afford the title compound (217 mg, 70 %).
¹H-NMR (CDCl₃) δ: 0.6-0.9 (6H, d), 1.2-1.6 (4H, m), 2.2-2.4 (4H, m), 2.55 (3H, s), 2.8 (2H, d), 5.3 (2H, brs), 6.25 (1H, t), 6.85 (1H, d), 7.27-7.4 (6H, m), 7.6-7.8 (2H, m), 8.0 (1 H, d), 8.5 5 (1 H, d).
MSm/z:517(M+1).

### Example 5

### R-1-(5-{3-[4-(4-Chloro-phenyl)-4-hydroxy-3,3-dimethyl-piperdin-1-yl]-propylidane}-5,11-dihydro-10-oxa-1-aza-dibenzo [a,d]cyclohepten-7-yl)-ethanone

To a solution of *R*-4-(4-chloro-phenyl)-3,3-dimethyl-piperdin-4-ol (100 mg, 0.417 mmol) in acetonitrile/water (4/1) was added potassium carbonate (57 mg, 0.413 mmol), followed by 1-[5-(3-bromo-propylidene)-5,11-dibydro-10-oxa-1-azadibenzo[a,*d*]cyclohepten-7-yl]-ethanone (100 mg, 0.279 mmol). The reaction mixture was stirred at 50 °C for 8 hours and then concentrated *in vacuo.* The resulting residue was treated with water and extracted with ethyl acetate. Solvent was evaporated from the combined dried (MgSO₄) organic extracts, and the residue was purified by column chromatography on silica gel using methylene chloridemethanol (96:4) to afford the titled compound (102 mg, 71 %).
¹H-NMR (CDCl₃) δ: 0.6-0.9 (6H, d), 1.2-1.6 (4H, m), 2.2-2.4 (4H, m), 2.55 (3H, s), 2.8 (2H, d), 5.3 (2H, brs), 6.25 (1H, t), 6.85 (1H, d), 7.27-7.4 (6H, m), 7.6-7.8 (2H, m), 8.0 (1H,d), 8.5 (1H, d).
MS m/z: 517 (M+ 1).

### Example 6

### Acetic acid 2-(5- {3-[4-(4-chloro-phenyl)-4-hydroxy-3,3-dimethyl-piperidin-1-yl]-propylidene} -5,11-dihydro-10-oxa-1-aza-dibenzo[a,d]cyclohepten-7-yloxy)-ethyl ester

To a solution of 5- {3-[4-(4-Cbloro-pheDyl}-4-hydroxy-3,3-dimethylpiperidin-1-yl]-propylidene}-5,11-dihydro-10-oxa-1-aza-dibenzo[a,d]cyclohepten-7-ol (64.5mg, 0.131mmo1) in *N*,*N*-dimethyformamide (2.5ml) was added sodium hydride (6 mg, 0.158mmol), 60% dispersion in mineral oil, then acetic acid 2-bromo-ethyl ester (17µl, 0.158mmol). The reaction stirred at room temperature overnight under a nitrogen atmosphere. The reaction was quenched the following day with water and diluted with ethyl acetate. The organic layer was washed twice with water, then brine. After the organic layer was dried over magnesium sulfate the solvent was distilled off under reduced pressure to give the title compound, 132mg (100%).
¹H-NMR(CDCl₃) δ:1.73-1.90(4H,m), 2.07-2.90(4H,s), 2.29-2.79(6H,m), 4.11-4.14(2H,bt), 4.36-4.40(2H,bt), 5.23-5.30 (2H,bs), 6.10-6.15(1H,t), 6.73-6.84(3H,m), 7.24-7.39(10H,m), 7.56-7.59(1H,dd), 8.47-8.50(1H,dd).
MS m/z: 577 (M)

### Example 7

### 4-(4-Chloro-phenyl)-1-{3-[7-(2-hydroxy-ethoxy)-11H- 10-oxa-1-aza-dibenzo[a,d] cyclohepten -5-ylidene]-propyl}-3,3-dimethyl-piperidin-4-ol

To a solution of 75.8mg (0.1314mmo1) Acetic acid 2-(5-{3-[4-(4-chlorophenyl)-4-hydroxy-3,3-dimethyl-piperidin-1-yl]-propylidene}-5,11-dihydro-10-oxa-1-aza-dibenzo[a,d]cyclohepien-7-yloxy)-ethyl ester (example 6) in ethanol (5ml) was added a 15% sodium hydroxide in water (2ml) solution. The reaction Was heated to reflux for 30 minutes, at which time the LC/MS showed it to be complete. After cooling to room temperature the reaction was worked up by diluting with ethyl acetate and washing with water and brine. The combined aqueous layers were back extracted with ethyl acetate. The combined organics were dried over magnesium sulfate and the solvent was distilled under reduced pressure. The residue was purified by reverse phase HPLC to give the title compound (42mg, 60%). ¹H-NMR(CDCl₃) δ: 0.821
(3H.s), 0.975(3H,s), 2.56-5.64(2H,bq), 2.68-2.74(1 H,bd), 2.80-3.08(5H,m), 3.30-3.38(1H,bd), 3.62-3.67(2H,t), 4.04-4.08 (2H,t), 5.19-5.32(2H,bs), 5.94-6.00(1H,t), 6.79-6.87(4H,m), 7.26-7.40(4H,m) 7.56-7.60(1H,bd), 8.35-8.37(1H,s), 8.50-8.53(1H,dd)
MS m/z: 535 (M)

### Example 8

### 4-(4-Chloro-phenyl)-1- {3-[7-(2-methoxy-ethoxy)-11 H-10-oxa-1-aza-dibenzo [a,d]cyclobepten-5-ylidene]-propyl}-3,3-dimethyl-piperidin-4-ol

The titled compound was prepared by following the procedure of Example 6, but replacing acetic acid 2-bromo-ethyl ester with 1-Bromo-2-methoxy-ethane. The residue was purified by reverse phase HPLC to give the title compound (27mg, 39%).
¹H-NMR(CDCl₃) δ: 0.79-.085(3H,s), 0.95-1.03(3H,s), 3.55-3.12(8H,m), 3-28-3.45(2H,m), 3.48(3H,bs), 3.70-3.78(2H, bs), 4.06-4.13(2H,bs), 5.18-5.28(2H,bs), 5.92-6.01 (1H,bt), 6.75-6.88(3H,m). 7.25-7.43(4H,m),-7.55-7.63(1H,bd), 8.48-8.55(2H, bd)
MS m/z: 549 (M)

### Example 9

### 5- {3-[4-(4-Chloro-phenyl)-4-hydroxy-3,3-dimethyl-piperidin-1-yl]-propylidene}-5,11 1-dihydro-10-oxa-1-aza-dibenzo[a, d]cyclohepten-7-ol

The titled compound was prepared-following the procedure in step 5 of Example 2, substituting 5-(3-bromo-propylidene)-5,11-dihydro-10-oxa-1-azadibenzo[a,d]cyclo-hepten-7-ol for 2-[5-(3-bromo-propylidene)-5,11-dihydro-10-oxa-1-aza-dibenzo[a,d]- cyclohepten-7-yl]-propan-2-ol and racemic piperidine for the S-piperidine.
¹H-NMR (CDCl₃, 300 MHz) δ : 0.74 (s, 3 H), 0.88 (s, 3 H), 1.45 (m, 1H), 2.20 - 2.54 (m, 7 H), 2.66 - 2.77 (m, 2 H), 5.27 (br s, 2 H), 6.11 (t, 1 H), 6.67 (dd, 1 H), 6.74 - 6.79 (m, 2 H), 7.25 - 7.31 (m, 3 H), 7.36 - 7.42 (m, 2 H), 7.59 (d, 1 H), 8.50 (dd, 1 H).
MS m/z: 491 (M + 1).

### Example 10

### 2-(5-{3-[4-(4-Chloro-phenyl)-4-hydroxy-3,3-dimethyl-piperidin-1-yl]-propylidene}-5,11-dihydro-10-oxa-1-aza-dibenzo [a,d]cyclohepten-7-yloxy)-2-methyl-propionic acid ethyl ester

To a cooled (0 °C) solution of 5-{3-[4-(4-Chloro-phenyl)-4-hydroxy-3,3-dimethyl-piperidin-1-yl]-propylidene}-5,11-dihydro-10-oxa-1-azadibenzo[a,d]cyclohepten-7-ol (250 mg, 0.51 mmol) in dimethylformamide (7 mL) was added sodium hydride (60 % dispersion in oil, 31 mg, 0.76 mmol). The resulting mixture was stirred at 0 °C forl0 min. Ethyl 2-bromo-2-methyl propionate (149 mg, 0.76 mmol) was added. Stirring at 0 °C was continued 10 min before the mixture was warmed to rt and stirred 4h. The reaction was then partitioned between ethyl acetate and water; the aqueous phase was back-extracted with additional ethyl acetate. The combined organic phases were washed thrice with water, brine and dried overmagnesium sulfate, filtered and concentrated. The crude residue was purified by silica gel chromatography (methylene chloride-95:5 methylene chloride:methanol gradient) to afford the titled compound (233 mg, 76 %). ¹H-NMR
(CDCl₃, 300 MHz) δ: 0.74 (s, 3 H), 0.87 (s, 3 H), 1.27 (t, 3 H), 1.41 (d, 1 H), 1.50 (br s, 1 H), 1.55 (s, 6 H), 2.18 - 2.56 (m, 7 H), 2.64-2.78 (m, 2 H), 4.24 (q, 2 H), 5.27 (brs, 1 H), 6.09 (t, 1 H), 6.67-6.76 (m, 2 H), 6.87 (d, 1 H), 7.25-7.31 (m, 2 H), 7.37 - 7.42 (m, 2 H), 7.57 (d, 2 H), 8.01 (s, 1 H), 8.d0 (d, 1 H).
MS m/z: 605 (M + 1).

### Example 11

### 4-(4-Chloro-phenyl)-1-{3-[7-(2-hydroxy-1,1-dimethyl-ethoxy)-11H-10-oxa-1-azadibenzo[a,d]cyclohepten-5-ylidene] -propyl}-3,3-dimethyl-piperidin-4-ol

To a cooled (0 °C) solution of 2-(5-{3-[4-(4-Chloro-phenyl)-4-hydroxy-3,3-dimethyl-piperidin-1-yl]-propylidene}-5,11-dihydro-10-oxa-1-azadibenzo[a,d]cyclohepten-7-yloxy)-2-methyl-propionic acid ethyl ester (117 mg, 0.193 mmol) in tetrahydrofuran (5 mL) was added lithium aluminum hydride (10 M solution in tetrahydrofuran, 0.39 mL, 0.39 mmol). The reaction was allowed to stir at 0 °C for 2 h. Excess hydride was quenched by the addition of a saturated aqueous potassium/sodium tartrate solution. The mixture was stirred at rt until two clear phases formed. The mixture was diluted with ethyl acetate and shaken. The extracts were dried over magnesium sulfate, filtered and concentrated. The crude residue was purified by silica gel chromatography (methylene chloride- 90:10 methylene chloride/methanol gradient) to afford the titled compound (102 mg, 94 %).
¹H-NMR (CDCl₃, 300 MHz) δ: 0.74 (s, 3H), 0.87 (s, 3 H), 1.25 (s, 6H), 1.41 (d, 1 H), 2.18 - 2.55 (m, 9 H), 2.65 - 2.79 (m, 2 H), 3.58 (d, 2 H), 5.20 - 5.40 (br s, 2 H), 6.12 (t, 1 H), 6.74 - 6.84 (m, 2 H), 6.92 (d, 1 H), 7.25 - 7.32 (m, 3 H), 7.36 - 7.42 (m, 2 H), 7.60 (dd, 1 H), 8.51 (dd, 1 H).
MS m/z: 563 (M+ 1).

### Example 12

### 2-(5-{3-[4-(4-Chloro-phenyl)-4-hydroxy-3,3-dimethyl-piperidin-1-yl]-propylidene}-5,11-dihydro-10-oxa-1-aza-dibenzo [a,d]cyclohepten-7-yloxy)-2-methyl-propionic acid

To a solution of 2-(5-{3-[4-(4-Chloro-phenyl)-4-hydroxy-3,3-dimethyl-piperidin-1-yl]-propylidene}-5,11-dihydro-10-oxa-1-aza-dibenzo[a,d]cyclohepten-7-yloxy)-2-methyl-propionic acid ethyl ester (158 mg, 0.261 mmol) in methanol (3 mL) was added sodium hydroxide (1 N in water, 1 mL). Following a slight exotherm, the mixture clarified. An additional aliquot of sodium hydroxide (1 N, 1 mL) was added after 1 h and the mixture was allowed to stir an additional 1 h at rt. The solvents were evaporated under reduced pressure and the residue dissolved in water (2.5 mL). The basic solution was neutralized (pH = 7) with hydrochloric acid (1 N) and the titled compound was precipitated and collected by suction filtration (86 mg, 57%). ¹H-NMR (CD₃OD, 300 MHz) δ: 0.80 (s, 3H), 0.91 (s, 3H), 1.47 (s, 6 H), 1.72 (d, I H), 2.56 - 2.72 (m, 2 H), 2.83 - 3.00 (m, 2 H), 3.15 - 3.24 (m, 3 H), 3.32 - 3.48 (m, 2 H), 5.18 (br s, 2 H), 6.04 (t, 1 H), 6.69 - 6.84 (m, 2 H), 6.97 (d, 1 H), 7.28 - 7.37 (m, 3H), 7.40 - 7.47 (m, 2 H), 7.67 (dd, 1 H), 8.42 (dd, 1 h).
MS m/z: 577 (M + 1).

### Example 13

### 2-(5-{3-[4-(4-Chloro-phenyl)-4-hydroxy-3,3-dimethyl-piperidin-1-yl]-propylidene}-5,11-dihydro-10-oxa-1-aza-dibenzo [a,d]cyclohepten-7-yloxy)-2-methyl-propionamide

To a solution of 2-(5-{3-[4-(4-Chloro-phenyl)-4-hydroxy-3.3-dimethyl-piperidin-1-yl]-propylidene}-5,11-dihydro-10-oxa-1-aza-dibenzo[a,d]cyclohepten-7-yloxy)-2-methyl-propionic acid (72 mg, 0.125 mmol) in dimethyl formamide (3 mL) was added 1-(3-dimethylaminopropyl)-3-ethylcarbodiimide hydrochloride (48 mg, 0.25 mmol), 1-hydroxybenzotriazole (34 mg, 0.25 mmol), and ammonium hydroxide(170 µL, 0.50 mmol) and triethylamine (100 µL). Stir at rt 48 h. The mixture was poured into chloroform and washed with water. The organic phase was washed with additional water_and brine; dried over magnesium sulfate, filtered and concentrated. The crude residue was purified by silica gel chromatography (methylene chloride- 90:10 methylene chloride/methanol gradient) to yield the titled compound (22 mg, 31 %). ¹H-NMR (CDCl₃, 300 MHz) δ: 0.74 (s, 3 H), 0.87 (s, 3 H), 1.42 (br d, 1 H), 1.49 (s, 6 H), 2.16 - 2.61 (m, 7 H), 2.63 - 2.78 (m, 2 H), 5.20 - 5.40 (br s, 2 H), 5.50 (br s, 1 H), 6.11 (t, 1 h), 6.68 - 6.82 (m, 3 H), 6.91 (dd, 1 H), 7.26 - 7.33 (m, 4 H), 7.36 - 7.43 (m, 2 H), 7.60 (dd, 1H), 8.51 (dd, 1 H).
MSm/z:576(M+1).

### Example 14

### (5-{3-[4-(4-Chloro-phenyl)-4-hydroxy-3,3-dimethyl-piperidin-1-yl]-propylidene}-5,11-dihydro-10-oxa-1-aza-dibenzo[a, d]cyclohepten-7-yloxy)-acetic acid methyl ester

5- {3-[4-(4-Chloro-phenyl)-4-hydroxy-3,3-dimethyl-piperidin-1-yl]-propylidene}-5,1 1-dihydro-10-oxa-1-aza-dibenzo[a,d]cyclohepten-7-ol (0.4 g, 0.814 mmol) was dissolved in dimethyl formamide (10 mL). Sodium hydride (0.029 g, 1.22 mmol, 1.5 equiv, 48 mg of a 60% suspension in mineral oil) was added at room temperature and gas evolution was visible. Methyl bromoacetate (0.187 g, 1.22 mmol, 116 uL) was added and the mixture was stirred at room temperature. After stirring overnight at room temperature, the reaction was quenched with water (10 mL). The resulting suspension was extracted with ethyl acetate (3x10 mL). The combined ethyl acetate extracts were dried over magnesium sulfate, filtered and concentrated *in vacuo* to give an oil. The crude oil was purified by silica gel chromatography (methylene chloride to 10 % methanol in methylene chloride gradient) to afford the title compound as a white solid (222 mg, 48%). ¹H-NMR (CDCl₃, 300 MHz) δ: 0.7 (s, 3H), 1.2 (s, 3H), 1.30 -1.70 (m, 3H), 2.62 - 3.51 (m, 9 H), 3.82 (s, 3H), 4.55 (s, 2H), 5.22 (br s, 1H), 5.88 (t, 1H), 6.85 (m, 2H), 7.15-733 (m, 6H), 7.62 (d, 1 H), 8.43 (d, 1H); MS m/z: 564 (M + 1)

### Example 15

### (5-{3-[4-(4-Chloro-phenyl)-4-hydroxy-3,3-dimethyl-piperidin-1-yl]-propylidene}-5,11-dihydro-10-oxa-1-aza-dibenzo[a, d]cyclohepten-7-yloxy)-acetic acid

(5- {3 [4-(4-Chloro-phenyl)- 4-hydroxy- 3,3-dimethyl-piperidin- 1-yl] -propylidene}- 5,11-dihydro- 10-oxa-1-aza-dibenzo[a,d]cyclohepten-7-yloxy)-acetic acid methyl ester (0.060 g, 0.107 mmol) was dissolved in methanol (1.5 mL). Sodium hydroxide (150 uL, 1 N stock solution) was added and the resulting mixture was stirred at room temperature. After stirring overnight at room temperature, the reaction mixture was concentrated *in vacuo* to give a yellow oil. The oil was suspended in water (2 mL) and then washed with ethyl acetate (3 x 3 mL). The aqueous layer was then acidified to pH = 2 and extracted with ethyl acetate (3 x 5 mL). The pooled organic layers were dried over magnesium sulfate, filtered and concentrated *in vacuo* to afford a white solid (55 mg, 94%).
¹H-NMR (CDCl₃, 300 MHz) δ: 0.8 (s, 3H), 0.92 (s, 3H), 1.12 - 1.31 (m, 6H), 2.68 (br d, 2H), 3.89 (m, 1H), 2.95 (d, 1H), 3.10- 3.36 (m, 2H), 4.45 (s, 1H), 5.20 (br s, 2H), 5.98 (t, 1H), 6.81 (m, 2H), 6.96 (d, 1H), 7.40 (d, 2H), 7.40 (m, 3H), 7.83 (d, 1H), 8.44 (d, 1H); MS m/z: 550 (M + 1)

### Example 16

### 2-(5-{3-[4-(4-Chloro-phenyl)-4-hydroxy-3,3-dimethyl-piperidin-1-yl]-propylidene}-5,11-dihydro-10-oxa-1-aza-dibenzo[a,d]cyclohepten-7-yloxy)actamide

The title compound was prepared by following the procedure of Example 14, but replacing methyl bromoacetate with bromoacetamide. This yielded 58 mg of the title compound after chromatography (52%). ¹H-NMR (CDCl₃, 300 MHz) δ: 0.5 (br s , 6H), 0.78 - 2.90 (m, 10H), 4.35 (s, 1H), 5.22 (br s, 2H), 5.74 (br s, 1H), 6.11 (br s , 1H), 6.55 (br s, 1H), 6.82 (m, 3H), 7.33 (d, 3H), 7.25 (d, 2H), 7.65, (d, 1H); 8.55 (d, 1H); MS m/z: 549 (M + 1)

### Example 17

4-(4-Chloro-phenyl)-1-{3-[7-(2-hydroxy-2-methyl-propoxy)-11H-10-oxa-1 -aza-dibenzo[a,d] cyclohepten-5-ylidene]-propyl}-3,3-dimethyl-piperidin-4-ol

(5-{3-[4-(4-Chloro-phenyl)-4-hydroxy-3,3-dimethyl-piperidin-1-yl]-propylidene}-5,11-dihydro-10-oxa-1-aza-dibenzo[a,d]cyclohepten-7-yloxy) -acetic acid methyl ester (52 mg, 0.0923 mmole) was dissolved in tetrahydrofuran (2 mL). The resulting solution was cooled to ice bath temperatures and methylmagnesium bromide (0.369 mmol, 264 uL of 1.4 M solution in toluene/tetrahydrofuran) was added dropwise. The reaction was stirred for 4 hrs at ice bath temperature and then warmed to room temperature overnight. Tetrahydrofuran was removed by concentration *in vacuo*. The resulting solid was dissolved in ethyl acetate (10 mL) and washed with saturated ammonium chloride (10 mL). The ethyl acetate extracts were dried over magnesium sulfate, filtered and concentrated *in vacuo* to give an oil. The crude oil was purified by silica gel chromatography (methylene chloride to 10 % methanol in methylene chloride gradient) to afford the title compound as a white solid (40 mg, 77 %).
¹H-NMR (CDCl₃, 300 MHz) δ: 0.92 (d, 6H), 1.34 (s, 6H), 1.62, (br s, 2H), 2.24 (m, 9H). 3.78 (s, 2H), 6.82 (m, 3H), 7.22 - 7.41 (m, 7H), 7.62 (d, 1H), 8.52 (d, 1H); MS m/z: 564 (M + 1)

### Example 18

3 -(5 - {3-[4-(4-Chloro-phenyl)-4-hydroxy-3,3-dimethyl- piperidin-1-yl]-propylidene)-5,11-dihydro-10-oxa-1-aza-dibenzo [*a,d*]cyclohepten-7-yl)propane- 1,2-diol

1-(3-(Allyl- 11*H*- 10-oxa- 1-aza-dibenzo [*a*, *d*] cydobepten- 5-ylidene) -propyl]- 4-(4-chloro-phenyl)- 3,3-dimethyl-piperidin-4-ol (0.044g, 0.08 mmol) was dissolved THF (2 mL) / H₂O (0.5 mL) and cooled ta 0°C. To the solution was added OsO₄ (1.07 mL-2.5% OsO₄ in t-butanol) and stirred at room temperature for 4h. The reaction was diluted with sat. sodium bisulfite, the organics were removed dried over Mg₂SO₄, filtered and evaporated *in vacuo,* then purified by Biotage flash chromatography (5% methanol/ 95% methylene chloride to 7.5% methanol/92.5% methylene chloride to 15% methanol/ 85% methylene chloride) to yield the title compound (0.025 g, 53 %).¹H-NMR (CDCl₃): δ 0.74 (ss 3H), 0.87 (s, 3H), 1.22 (d, 1H), 1.41 (s, 1H), 1.44 (d, 1H), 2.26-2.77 (m, 9H), 3.53 (d, 1H), 3.70 (dd, 1 H), 3.93 (s, 1H), 5.30 (bs, 2H), 6.14 (t, 1H), 6.79 (d, 1H), 7.02 (d, 1H), 7.16 (s, 1H), 7.27 (d, 3H), 7.37 (d, 2H), 7.58 (d, 1H), 8.48 (dd, 1H). ESI-MS m/z: 515 (M + 1), retention time 1.72.

### Example 19

### 4-(4-Chloro-phenyl)-1-{3-[7-(1-hydroxy-1-methyl-ethyl)-1-oxy-11H-10-oxa-1-azadibenzo[a,d]cydobepten-5-ylidene] -propyl}-3,3-dimethyl-piperidin-4-ol

### Step 1: 1-[5-(3-Bromo-propylidene)-1-oxy-5,11-dihydro-10-oxa-1-azadibenzo[a,d]cyclohepten-7-yl]-ethanone

A solution of 1-[5-(3-Bromo-propylidene)-5,1 1-dihydro-10-oxa-1-azadibenzo[a,d]cyclohepten-7-yl]-ethanone (1.5 g, 4.2 mmol) in CH₂Cl₂ (10 mL) was treated with m-chloroperbenzoic acid (77%, 1.2 g, 5.0 mmol). The mixture was allowed to stir at rt for 12 hr, then quenched with aqueous brine and extracted with CH₂Cl₂ (3x30 mL). The combined organics were dried over sodium sulfate and evaporated *in vacuo.* The resulting brown foam, 1.5 g (95%), was carried on to the next step crude. ESI-MS m/z: 374 [M + 1].

### Step.2: 1-(5-{3-[4-(4-Chloro-phenyl)4-hydroxy-3,3-dimethyl-piperidin-1-yl]-propylidene}-1-oxy-5,11-dihydro-10-oxa-1-aza-dibenzo[a,d]cyclohepten-7-yl)ethanone

Asolutionofl-[5-(3-Bromo-propylidene)-1-oxy-5,11-dihydro-10-oxa-1-aza-dibenzo[a,d]cyclohepten-7-yl]-ethanone (1.4 g, 3.8 mmol) in acetonitrile/water (12 mL/3 mL) was treated with 4-(4-Chloro-phenyl)-3,3-dimethyl-piperidin-4-ol (1.0 g, 4.2 mmol) and potassium carbonate (1.1 g, 7.6 mmol). The suspension was stirred at rt for 72 hr, and evaporated *in vacuo.* The residue was purified by silica gel chromatography (ethyl acetate→87:10:3 ethyl acetate/ methanol/triethylamine) to yield 1.2 g (58%) of the title compound, ESI-MS m/z: 533 [M + 1].

### Step 3:4-(4-Chloro-phenyl)-1-{3-[7-(1-hydroxy-1-methyl-ethyl)-1-oxy-11H-10-oxa-1-aza-dibenzo[a,d]cyclohepten-5-ylidene]-propyl}-3,3-dimethyl-piperidin-4-ol

Asolutionofl-(5-{3-[4-(4-Chloro-phenyl)-4-hydroxy-3,3-dimethyl-piperidin-1-yl]-propylidene}-1-oxy-5,11-dihydro-10-oxa-1-azadibenzo[a,d]cyclobepten-7-yl)-ethanone (0.43 g, 0.81 mmol) in THF (3 mL) was treated with methylmagnesium bromide (1.4M, 0.74 mL, 1.0 mmol). The solution was stirred at rt for 48 hr, and quenched with brine. The aqueous residue was extracted with ethyl acetate (3x10 mL), and the combined organics were evaporated *in vacuo.* The residue was purified by silica gel chromatography (ethyl acetate→ 87:10:3 ethyl acetate/methanol/triethylamine) to yield 0.10g (25%) of the title compound, ¹H-NMR (CDCl₃) ∂: 0.6 (3H, s), 0.9 (3H, s), 1.55 (6H, s), 1.99-2.80 (10H, m), 5.30 (2H, brs), 6.18 (1 H, br), 6.89 (1 H, m), 7.25-7.45 (7H, m); 7.50 (1 H, m), 8.55 (1 H, d).
ESI-MS m/z: 549 [M+1].

### Example 20

### Isopropyl-carbamic acid 5-{3-[4-(4-chloro-phenyl)-4-hydroxy-3,3-dimethylpiperidin-1-yl]-propylidene}-5,11-dihydro-10-oxa-1-aza-dibenzo[a,d]cyclohepten-7-yl ester

S- {3-[4-(4-Chloro-phenyl)- 4-hydroxy- 3,3-dimethyl-piperidin- 1-yl] -propylidene}- 5,11-dihydro- 10-oxa-1-aza-dibenzo[a,d]cyclohepten-7-ol (0.27 mmol, 1.0 equiv) was dissolved in tetrahydrofuran and treated with triethylamine (1.50 equiv) and isopropyl isocyanate (0.4 mmol, 1.50 equiv). The resulting mixture was heated to 50 °C for 18 h. The reaction mixture was concentrated and crude product was purified on silica using gradient elution from ethyl acetate (100%) and ethyl acetate/methanol (5%). ¹H-NMR (CDCl₃) δ: 0.70 (3H, s), 0.90 (3H, s), 1.1 (3H, d), 1.25 (3H, d), 1.4 (1H, m), 1.6 (1 H, m), 2.30-2.50 (8H, m), 2.70 (2H, m), 3.80-4.00 (3H, m), 3.55 (3H, s), 4.85 (1 H, m), 5.30 (2H, br, s), 6.20 (1 H, t), 6.80-7.00 (2H, dd), 7.20-7.45 (6H, m), 7.60 (1 H, dd), 8.50 (1 H, dd). ESI-MS m/z : 576 (M + 1), UV retention time: 1.58 min.

### Example 21

### 4-(4-Chloro-phenyl)-3,3-dimethyl-1-{3-[7-(2-morpholin-4-yl-ethoxy)-11H-10-oxa-1-aza-dibenzo[a,d]cyclohepten-5-ylidene]-propyl}-piperidin-4-ol

To a solution of 5-{3-[4-(4-chloro-phenyl)-4-hydroxy-3,3-dimethyl-piperidin-1-yl]-propylidene}-5,11-dihydro-10-oxa-1-aza-dibenzo[a,d]cyclohepten-7-ol (0.15 g, 0.28 mmol) in DMF (1.5 mL) was added NaH (0.034 g, 0.84 mmol) and stirred for 20 min. at room temperature. 4-(2-Chloro-ethyl)-morpholine HCl (0.063 g, 0.34 mmol) was added and the solution was heated to 50 °C for 16h. The reaction was quenched with water and extracted with ethyl acetate (3x). The organics were combined, dried over Mg₂SO₄, filtered and concentrated, then purified by Biotage flash chromatography (5-10% methanol/dichloromethane) to yield the title compound (0.050 g). ¹H-NMR (MeOD) δ: 0.73 (3H, s), 0.85 (3H, s), 2.39-2.90 (11 H, m), 3.29-3.31 (3H, m), 3.69-3.72 (4H, m), 4.1 (2H, t), 5.20 (2H, br s), 6.18 (1 H, t), 6.74-6.79 (2H, m), 6.89 (1H, d), 7.27-7.30 (2H, m), 7.43-7.47 (3H, m), 7.79 (1H, dd), 8.46 (1H, dd). ESI-MS m/z : 604 (M), UV retention time: 1.20 min.

## Claims

1. A compound having the formula: 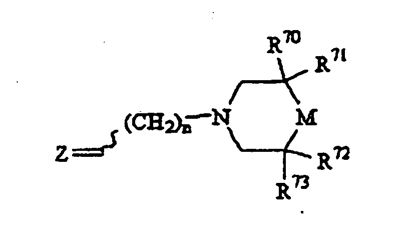 or physiologically acceptable salt thereof, wherein:
n is one to four;
M is >NR² or >CR¹R²;
R¹ is-H, -OH, -N₃, a halogen, an aliphatic group, a substituted aliphatic group, an aminoalkyl group,-O-(aliphatic group), -O-(substituted aliphatic group), -SH, -S-(aliphatic group), -S-(substituted aliphatic group), -OC(O)
-(aliphatic group), -O-C(O)-(substituted aliphatic group), -C(O)O-(aliphatic group), -C(O)O-(substituted aliphatic group), -COOH, -CN, -CO-NR³R⁴ or -NR³R⁴
R² is-OH, a halogen, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -0-(substituted or unsubstituted aromatic group), -0-(substituted or unsubstituted aliphatic group), -C(O)-(substituted or unsubstituted aromatic group) or -C(O)-(substituted or unsubstituted aliphatic group);
R³, R⁴, R⁵ and R⁶ are independently-H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclie or heterocyclic ring;
R⁷⁰ and R⁷¹ are independently -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C (O)O-(substituted aliphatic group), -COOH, -CN, -CO-NR³R⁴, -NR³R⁴, an acyl group, a substituted acyl group, a benzyl group, a substituted benzyl group, a non-aromatic heterocyclic group, a substituted non-aromatic heterocyclic group or -O-(substituted or unsubstituted aromatic group);
R⁷² and R⁷³ are independently -OH, -N₃, a halogen, an aliphatic group, a substituted aliphatic group, an aminoallcyl group, -O-(aliphatic group), -O-(substituted aliphatic group), -SH, -S-(aliphatic group), -S-(substituted aliphatic group), -O-C(O)-(aliphatic group), -O-C(O)-(substituted aliphatic group), -C(O)O-(aliphatic group), -C (O)O-(substituted aliphatic group), -COOH, -CN, -CO-NR³R⁴, -NR³R⁴, an acyl group, a substituted acyl group, a benzyl group, a substituted benzyl group, a non-aromatic heterocyclic group, a substituted non-aromatic heterocyclic group or -O-(substituted or unsubstituted aromatic group);
Z is 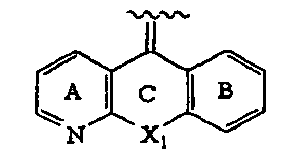
X₁ is-CH₂-O-, -O-CH₂-, -S-, -CH₂-, -CH₂-CH₂-, -CH₂-S-, -S-CH₂-, -NR_{c}-CH₂-, -CH₂-NR_{c}-, -SO-CH₂-, -CH₂-SO-, -S(O)₂-CH₂, -CH₂-S(O)₂-, -CH=CH-, -NR_{c}-CO-, a bond, -O-, or -CO-NR_{c}-;
R_{c} is -H, an aliphatic group, a substituted aliphatic group, an aromatic group, a substituted aromatic group, a benzyl group or a substituted benzyl group;
Rings A and B are independently unsutistituted or substituted;
said acyl group is an aliphatic carbonyl, aromatic carbonyl, aliphatic sulfonyl or aromatic sulfonyl; said aliphatic group is a C₁-C₆ alkyl, alkenyl or alkynyl;
said aromatic group is selected from the group consisting of phenyl, 1-naphthyl, 2-naphthyl, 1-anthracyl, 2-anthracyl, *N*-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl, 5-oxazolyl, tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 1-isoquinolinyl, 3-quinolinyl, 1-isoindolyl, 3-isoindolyl, acridinyl, 3-benzisoxazolyl, benzocyclopentyl and benzocyclohexyl;
said non-aromatic heterocyclic group is a five to eight-membered non-aromatic ring which contains one or more heteroatoms independently selected from the group consisting of nitrogen, oxygen and sulfur;
said substituted aliphatic group is substituted with one or more substituents selected from the group consisting of oxo group, epoxy group, non-aromatic heterocyclic ring, benzyl group, substituted benzyl group, aromatic group or substituted aromatic group electron withdrawing group, halo, azido, -CN, -CON R²⁴R²⁵, -NR²⁴R²⁵, -OS (O)₂NR²⁴R²⁵, -S(O)₂NR²⁴R²⁵, -SO₃H, guanidino, oxalo, -C(=NR⁶⁰NR²¹R²², =NR⁶⁰, -(O)ᵤ-(CH₂)ₜC(O)OR²⁰,
-(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -O-(aliphatic group), -Q-(substituted aliphatic group), -Q-(aryl), -O-(aromatic group), -O-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said substituted non-aromatic heterocyclic ring is substituted with one or more substituents selected from the group consisting of=O, =S, electron withdrawing group, halo, azido, -CN, -CONR²⁴R²⁵, -NR²⁴R²⁵, -OS (O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)₁-C(O)OR²⁰, -(O)ᵤ-(CH₂)ₜ-OC(O)R²⁰, -(O)ᵤ-(CH)₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -O-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said substituted aromatic group, substituted benzyl group, Ring A when substituted and Ring B when substituted, are substituted with one or more substituents selected from the group consisting of electron withdrawing group, aliphatic group, substituted aliphatic group, aromatic group, substituted aromatic group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜC(O)OR²⁰, -(O)ᵤ-(CH₂)ₜ-OC(O)R²⁰, -(O)ᵤ-(CH₂)ₜ-C(O)-NR²¹R²² -(O)ᵤ-(CH₂)ₜ-NHC(O) O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
Q is -O-, -S-, -S(O)-, -S(O)₂-, -OS(O)₂-, -C(O)-, -OC(O)-, -C(O)-, -C(O)C(O)-O-, -O-C(O)C(O)-, -NHC(O)-, -OC (O)NH-, -NH-C(O)-NH-, -S(O)₂NH-, -NHS(O)₂-, -C(NR²³)NHNH-, -NHNHC(NR²³), -NR²⁴C(O)- or-NR²⁴S(O)₂-; R²⁰, R²¹ and R²² are independently -H, an aliphatic group, an aromatic group, a non-aromatic heterocyclic group, -NHC(O)-O-(aliphatic group),
-NHC(O)-O-(aromatic group) or -NHC(O)-O-(non-aromatic heterocyclic group) or R²¹ and R²², taken together with the nitrogen atom to which they are bonded, can form a substituted or unsubstituted non-aromatic heterocyclic ring;
R²³ is -H, an aliphatic group, a benzyl group, an aryl group or non-aromatic heterocyclic group;
R²⁴ and R²⁵ are independently -H, an aliphatic group, a substituted aliphatic group, a benzyl group, an aryl group, non-aromatic heterocyclic group or R²⁴ and R²⁵ taken together with the nitrogen atom to which they are bonded can form a substituted or unsubstituted non-aromatic heterocyclic ring.
R⁶⁰ is a -H, -OH, -NH₂, an aromatic group or a substituted aromatic group.
t is zero to three;
u is zero or one; and
p is one to five.

2. The compound of Claim 1 wherein Ring A is unsubstituted and B is substituted para to the carbon atom of ring B that is bonded to X₁ in ring C, and Z is represented by the structural formula: 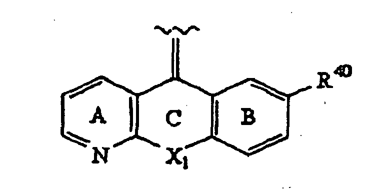 wherein R⁴⁰ is -OH, -COOH, -NO₂, halogen, aliphatic group, substituted aliphatic group, an aromatic group, a substituted aromatic group, -NR²⁴R²⁵, -CONR²⁴R²⁵, -NR²⁴C(O)-(aliphatic group), -NR²⁴C(O)-(substituted aliphatic group), -NR²⁴S(O)₂-(aliphatic group), -NR²⁴S(O)₂-(substituted aliphatic group), -C(O)O-(aliphatic group), -C(O) O-(substituted aliphatic group), -C(O)-(aliphatic group), -C(O)-(substituted aliphatic group), -O-(aliphatic group), -0-(substituted aliphatic group), -O-(aromatic group), -O-(substituted aromatic group), an electron withdrawing group, -(O)ᵤ-(CH₂)ₜ-C(O)OR²⁰, -(O)ᵤ-(CH₂)ₜOC(O)R²⁰, -(O)ᵤ-(CH₂)ₜ-C(O)-NR²¹R²² or -(O)ᵤ-(CH₂)ₜ-NHC(O)O-R²⁰; R²⁰, R²¹ or R²² are independently -H, an aliphatic group, a substituted aliphatic group, an aromatic group, a substituted aromatic group or a non-aromatic heterocyclic group; or
R²¹ and R²², taken together with the nitrogen atom to which they are bonded, form a non-aromatic heterocyclic ring; R²⁴ and R²⁵ are independently -H, an aliphatic group or a substituted aliphatic group;
u is zero or one; and
t is an integer from zero to 3.

3. The compound of Claim 2 wherein
M is >CR¹R²;
R¹ is -H or -OH; and
R² is a substituted aromatic group, wherein said substituted aromatic group is 4-halophenyl.

4. The compound of Claim 3 wherein said 4-halophenyl is selected from the group consisting of 4-chlorophenyl, 4-bromophenyl and 4-fluorophenyl.

5. The compound of Claim 3 wherein X₁ is -CH₂-O-.

6. The compound of Claim 2 wherein at least one of R⁷⁰, R⁷¹, R⁷² and R⁷³ is an aliphatic group or a substititued aliphatic group; wherein
said aliphatic group is a C₁-C₆ allcyl and said substituted aliphatic group is a C₁-C₆ alkyl substituted with a substitutent selected from the group consisting of-OH, -(O)ᵤ-(CH₂)ₜ-C(O)OR₂₀ and -O-(aliphatic group); t is zero to three;
u is zero or one; and
R²⁰ is C₁-C₆ alkyl.

7. The compound of Claim 6 wherein
R⁷⁰ and R⁷¹ are both -H; and
R⁷² and R⁷³ are independently selected from the group consisting of C₁-C₆ alkyl and substititued C₁-C₆ alkyl.

8. The compound of Claim 7 wherein R⁷² is -CH₃.

9. A compound having the formula: 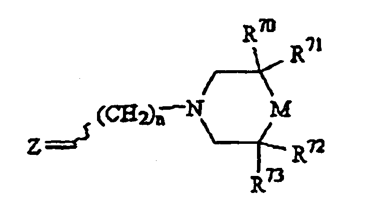 or physiologically acceptable salt thereof, wherein:
n is one to four;
M is >CR¹R²_{;}
R¹ is -OH;
R² is 4-halophenyl;
R⁷⁰ and R⁷¹ are -H, and R⁷² and R⁷³ are -CH₃; or
R⁷⁰ and R⁷¹ are -CH₃, and R⁷² and R⁷³ are -H;
Zis 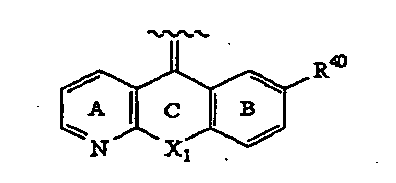 X₁ is -CH₂-O-; and
R⁴⁰ is selected from the group consisting of: 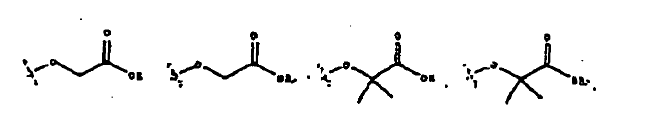 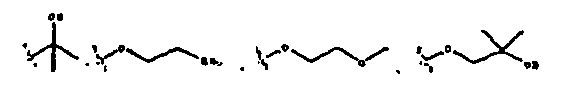 and

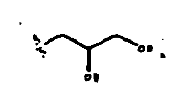

10. The compound of Claim 9 wherein said 4-halophenyl is selected from the group consisting of 4-chlorophenyl, 4-bromophenyl and 4-fluorophenyl

11. The compound of Claim 10 wherein R⁷⁰ and R⁷¹ are -H, R⁷² and R⁷³ are -CH₃, n is two, and the compound has the structure:

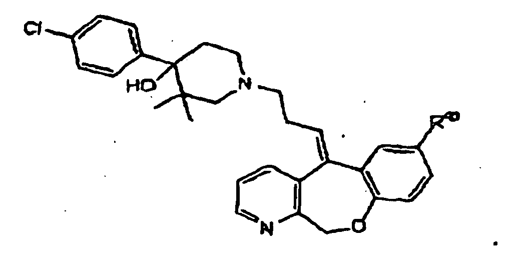

12. The compound of Claim 11 wherein R⁴⁰ is

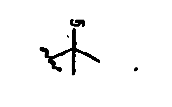

13. A compound having the structure: 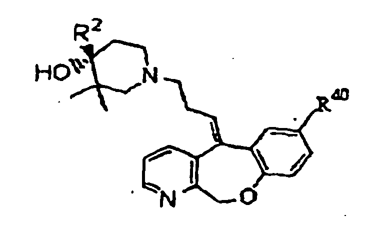 or a physiologically acceptable salt thereof, wherein
R² is 4-halophenyl; and
R⁴⁰ is selected from the group consisting of: 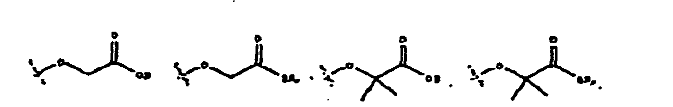 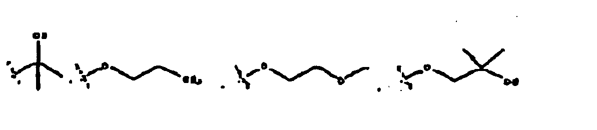 and

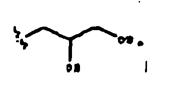

14. The compound of Claim 13 wherein R⁴⁰ is

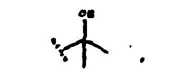

15. The compound of Claim 13 wherein R² is selected from the group consisting of 4-chlorophenyl, 4-bromephenyl and 4-fluorophenyl.

16. The compound of Claim 15 wherein R² is 4-chlorophenyl.

17. A pharmaceutical composition comprising a compound according to any one of Claims 1 to 16 and a physiologically acceptable carrier.

18. A compound according to any one of Claims 1 to 16 for use in therapy.

19. Use of a compound according to any one of Claims 1 to 16 for the manufacture of a medicament for treating a disease associated with aberrant leukocyte recruitment, aberrant leukocyte activation or aberrant leukocyte recruitment and activation.

20. Use of Claim 19, wherein said disease is selected from the group consisting of arthritis, atherosclerosis, arteriosclerosis, restenosis, ischemia/reperfusion injury, diabetes mellitus, psoriasis, multiple sclerosis, inflammatory bowel diseases, rejection of a transplanted organ or tissue, graft versus host disease, allergy and asthma.

21. Use of a compound according to any one of Claims 1 to 16 for the manufacture of a medicament for treating a chronic inflammatory disease.

22. Use of a compound according to any one of Claims 1 to 16 for the manufacture of a medicament for treating multiple sclerosis.

23. Use of a compound according to any one of Claims 1 to 16 for the manufacture of a medicament for treating arthritis.

24. Use of Claim 23, wherein said arthritis is rheumatoid arthritis.

## Patentansprüche

1. Verbindung der Formel: 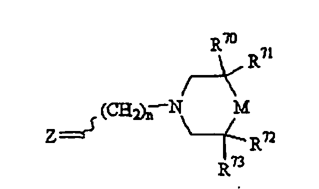 oder ein physiologisch annehmbares Salz davon, worin bedeuten:
n ist eins bis vier;
M ist >NR² oder >CR¹R²;
R¹ ist -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(substituierte aliphatische Gruppe), -COOH, -CN, -CO-NR³R⁴ oder NR³R⁴;
R² ist -OH, ein Halogen, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe, -O-(substituierte oder unsubstituierte aromatische Gruppe), -O-(substituierte oder unsubstituierte aliphatische Gruppe), -C(O)-(substituierte oder unsubstituierte aromatische Gruppe) oder -C(O)-(substituierte oder unsubstituierte aliphatische Gruppe);
R³, R⁴, R⁵ und R⁶ sind unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe; oder
R¹ und R², R³ und R⁴, oder R⁵ und R⁶ bilden zusammen mit dem Atom, an das sie gebunden sind, einen substituierten oder unsubstituierten nicht-aromatischen carbocyclischen oder heterocyclischen Ring;
R⁷⁰ und R⁷¹ sind unabhängig von einander -H, -OH, -N₃, ein Halogen, eine aliphatische Gruppe, eine substituierte aliphatische Gruppe, eine Aminoalkylgruppe, eine -0-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(subsituierte aliphatische Gruppe), -COOH, -CN, -CO-NR³R⁴, -NR³R⁴, eine Acylgruppe, eine substituierte Acylgruppe, eine Benzylgruppe, eine substituierte Benzylgruppe, eine nichtaromatische heterocyclische Gruppe, eine substituierte nicht-aromatische heterocyclische Gruppe oder eine -O-(substituierte oder unsubstituierte aromatische Gruppe);
R⁷² und R⁷³ sind unabhängig von einander -OH, -N₃, ein Halogen, eine aliphatische Gruppe, eine substituierte aliphatische Gruppe, eine Aminoalkylgruppe, -0-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(subsituierte aliphatische Gruppe), -COOH, -CN, -CO-NR³R⁴, -NR³R⁴, eine Acylgruppe, eine substituierte Acyl- gruppe, eine Benzylgruppe, eine substituierte Benzylgruppe, eine nicht-aromatische heterocyclische Gruppe, eine substituierte nicht-aromatische heterocyclische Gruppe oder eine -O-(substituierte oder unsubstituierte aromatische Gruppe);
Z ist 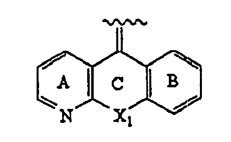 X₁ ist -CH₂-O-, -O-CH₂-, -S-, -CH₂-, -CH₂-CH₂-, -CH₂-S-, -S-CH₂-, -NR_{c}-CH₂-, -CH₂-NR_{c}-, -SO-CH₂-, -CH₂-SO-, -S(O)₂-CH₂-, -CH₂-S(O)₂-, -CH=CH-, -NR_{c}-CO-, eine Bindung, -O- oder -CO-NR_{c}; R_{c} ist -H, eine aliphatische Gruppe, eine substituierte aliphatische Gruppe, eine aromatische Gruppe, eine substituierte aromatische Gruppe, eine Benzylgruppe oder eine substituierte Benzylgruppe;
Ringe A und B sind unabhängig von einander unsubstituiert oder substituiert;
die Acylgruppe ist ein aliphatisches Carbonyl, aromatisches Carbonyl, aliphatisches Sulfonyl oder aromatisches Sulfonyl;
die aliphatische Gruppe ist ein C₁-C₆-Alkyl, Alkenyl oder Alkinyl;
die aromatische Gruppe ist ausgewählt aus der Gruppe bestehend aus Phenyl, 1-Naphthyl, 2-Naphthyl, 1-Anthracyl, 2-Anthracyl, N-Imidazolyl., 2-Imidazolyl, 4-Imidazolyl, 5-Imidazolyl, 2-Thienyl, 3-Thienyl, 2-Furanyl, 3-Furanyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, 3-Pyrazolyl, 4-Pyrazolyl, 5-Pyrazolyl, 2-Pyrazinyl, 2-Thiazolyl, 4-Thiazolyl, 5-Thiazolyl, 5-Tetrazolyl, 2-Oxazolyl, 4-Oxazolyl, 5-Oxazolyl, Tetrahydronaphthyl, 2-Benzothienyl, 3-Benzothienyl, 2-Benzofuranyl, 3-Benzofuranyl, 2-Indolyl, 3-Indolyl, 2-Chinolinyl, 3-Chinolinyl, 2-Benzothiazolyl, 2-Benzooxazolyl, 2-Benzimidazolyl, 1-Isochinolinyl, 3-Chinolinyl, 1-Isoindolyl, 3-Isoindolyl, Acridinyl, 3-Benzisoxazolyl, Benzocyclopentyl und Benzocyclohexyl;
die nicht-aromatische heterocyclische Gruppe ist ein 5- bis 8-gliedriger nicht-aromatischer Ring, der ein oder mehrere Heteroatome enthält, unabhängig von einander ausgewählt aus der Gruppe bestehend aus Stickstoff, Sauerstoff und Schwefel;
die substituierte aliphatische Gruppe ist mit einem oder mehreren Substituenten substituiert, ausgewählt aus der Gruppe bestehend aus Oxogruppe, Epoxygruppe, nicht-aromatischer heterocyclischer Ring, Benzylgruppe, substituierte Benzylgruppe, aromatische Gruppe oder substituierte aromatische Gruppe, Elektronen-abziehende Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ(CH₂)ₜ-OC(O)R²⁰, -(O)ᵤ(CH₂)ₜ(O)-Nr²¹R²²_{,} -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe) und -Q-(CH₂)ₚ-(nicht-aromatische heterocyclische Gruppe);
der substituierte nicht-aromatische heterocyclische Ring ist mit einem oder mehreren Substituenten substituiert ausgewählt aus der Gruppe bestehend aus =O, =S, Elektronen-abziehende Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ-(CH₂)ₜOC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe) und -Q-(CH₂)ₚ-(nicht-aromatische heterocyclische Gruppe);
die substituierte aromatische Gruppe, substituierte Benzylgruppe, Ring A, wenn subsituiert, und Ring B, wenn substituiert, sind mit einem oder mehreren Substituenten substituiert, ausgewählt aus der Gruppe bestehend aus einer Elektronen-abziehenden Gruppe, einer aliphatischen Gruppe, substituierten aliphatischen Gruppe, aromatischen Gruppe, subsituierten aromatischen Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ₋(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe) und -Q-(CH₂)ₚ-(nicht-aromatische heterocyclische Gruppe);
Q ist -O-, -S-, -S(O)-, -S(O)₂-, -OS(O)₂-, -C(O)-, -OC(O)-, -C(O)O-, -C(O)C(O)-O-, -O-C(O)C(O)-, -NHC(O)-, -OC(Q)NH-, -NH-C(O)-NH-, -S(O)₂NH-, -NHS(O)₂-, -C(NR²³)NHNH-, -NHNHC(NR²³)-, -NR²⁴C(O)- oder -NR²⁴S(O)₂-;
R²⁰, R²¹ und R²² sind unabhängig von einander -H, eine aliphatische Gruppe, eine aromatische Gruppe, eine nicht-aromatische heterocyclische Gruppe, -NHC(O)-O-(aliphatische Gruppe), -NHC(O)-O-(aromatische Gruppe) oder -NHC(O)-O-(nicht-aromatische heterocyclische Gruppe), oder R²¹ und R²² können zusammen mit dem Stickstoffatom, an das sie gebunden sind, einen substituierten oder unsubstituierten nicht-aromatischen heterocyclischen Ring bilden;
R²³ ist -H, eine aliphatische Gruppe, eine Benzylgruppe, eine Arylgruppe oder eine nicht-aromatische heterocyclische Gruppe;
R²⁴ und R²⁵ sind unabhängig von einander -H, eine aliphatische Gruppe, eine substituierte aliphatische Gruppe, eine Benzylgruppe, eine Arylgruppe, eine nicht-aromatische heterocyclische Gruppe, oder R²⁴ und R²⁵ können zusammen mit dem Stickstoffatom, an das sie gebunden sind, einen substituierten oder unsubstituierten nicht-aromatischen heterocyclischen Ring bilden;
R⁶⁰ ist -H, -OH, -NH₂, eine aromatische Gruppe oder eine substituierte aromatische Gruppe;
t ist Null bis Drei;
u ist Null oder Eins; und
p ist Eins bis Fünf.

2. Verbindung nach Anspruch 1, worin Ring A unsubstituiert ist und Ring B zum Kohlenstoffatom von Ring B, das an X₁ im Ring C gebunden ist, para-substituiert ist, und Z durch die Strukturformel repräsentiert wird: 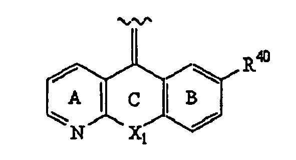 worin R⁴⁰ -OH, -COOH, -NO₂, Halogen, aliphatische Gruppe, substituierte aliphatische Gruppe, eine aromatische Gruppe, eine substituierte aromatische Gruppe, -NR²⁴R²⁵, -CONR²⁴R²⁵, - NR²⁴C(O)-(aliphatische Gruppe), -NR²⁴C(O)-(substituierte aliphatische Gruppe), -NR²⁴S(O)₂-(aliphatische Gruppe), -NR²⁴S(O)₂-(substituierte aliphatische Gruppe), -C(O)O-(aliphatische Gruppe), -C(O)O-(substituierte aliphatische Gruppe), -C(O)-(aliphatische Gruppe), -C(O)-(substituierte aliphatische Gruppe), -O-(aliphatische Gruppe), -O-(substituierte aliphatische Gruppe), -O-(aromatische Gruppe), -O-(substituierte aromatische Gruppe), eine Elektronen-abziehende Gruppe, -(O)ᵤ-(CH₂)ₜ-C(O)OR²⁰, -(O)ᵤ-(CH₂)ₜ-OC(O)R²⁰, -(O)ᵤ-(CH₂)ₜC(O)-NR²¹R²², oder -(O)ᵤ-(CH₂)ₜ-NHC(O)O-R²⁰ ist;
R²⁰, R²¹ oder R²² unabhängig von einander -H, eine aliphatische Gruppe, eine substituierte aliphatische Gruppe, eine aromatische Gruppe, eine substituierte aromatische Gruppe oder eine nicht-aromatische heterocyclische Gruppe sind; oder
R²¹ und R²² zusammen mit dem Stickstoffatom, an das sie gebunden sind, einen nicht-aromatischen heterocyclischen Ring bilden;
R²⁴ und R²⁵ unabhängig von einander -H, eine aliphatische Gruppe oder eine substituierte aliphatische Gruppe sind;
u ist Null oder Eins; und
t ist eine ganze Zahl von Null bis Drei.

3. Verbindung nach Anspruch 2, worin bedeuten:
M ist >CR¹R²;
R¹ ist -H oder -OH; und
R² ist eine substituierte aromatische Gruppe, worin die substituierte aromatische Gruppe 4-Halogenphenyl ist.

4. Verbindung nach Anspruch 3, worin das 4-Halogenphenyl ausgewählt ist aus der Gruppe bestehend aus 4-Chlorphenyl, 4-Bromphenyl und 4-Fluorphenyl.

5. Verbindung nach Anspruch 3, worin X₁ -CH₂-O- ist.

6. Verbindung nach Anspruch 2, worin mindestens einer der Reste R⁷⁰, R⁷¹, R⁷² und R⁷³ eine aliphatische Gruppe oder eine substituierte aliphatische Gruppe ist; worin
die aliphatische Gruppe C₁-C₆-Alkyl ist, und die substituierte aliphatische Gruppe C₁-C₆-Alkyl ist, das substituiert mit (einem) Substituenten ausgewählt aus der Gruppe bestehend aus -OH, -(O)ᵤ-(CH₂)ₜC(O)OR₂₀ und -O-(aliphatische Gruppe) substituiert ist.
t ist Null bis Drei;
u ist Null oder Eins; und
R²⁰ ist C₁-C₆-Alkyl.

7. Verbindung nach Anspruch 6, worin
R⁷⁰ und R⁷¹ beide -H sind; und
R⁷² und R⁷³ unabhängig von einander ausgewählt sind aus der Gruppe bestehend aus C₁-C₆-Alkyl und substituiertem C₁-C₆-Alkyl.

8. Verbindung nach Anspruch 7, worin R⁷² -CH₃ ist.

9. Verbindung der Formel: 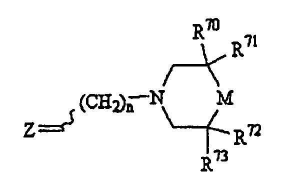 oder ein physiologisch annehmbares Salz davon, worin bedeuten:
n ist Eins bis Vier;
M ist >CR¹R²;
R¹ ist -OH;
R² ist 4-Halogenphenyl;
R⁷⁰ und R⁷¹ sind -H, und R⁷² und R⁷³ sind -CH₃; oder
R⁷⁰ und R⁷¹ sind -CH₃, und R⁷² und R⁷³ sind -H; Z ist 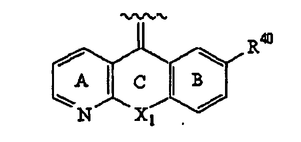
X₁ ist -CH₂-O-; und
R⁴⁰ ist ausgewählt aus der Gruppe bestehend aus: 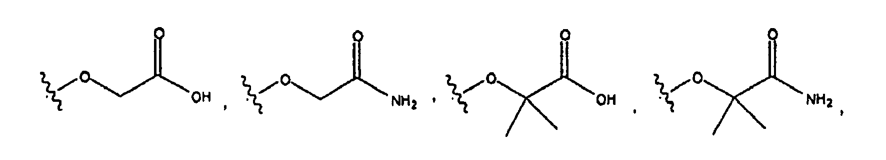 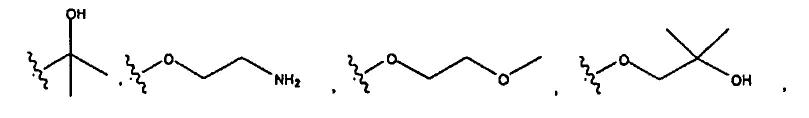
und

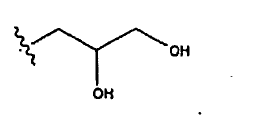

10. Verbindung nach Anspruch 9, worin das 4-Halogenphenyl ausgewählt ist aus der Gruppe bestehend aus 4-Chlorphenyl, 4-Bromphenyl und 4-Fluorphenyl.

11. Verbindung nach Anspruch 10, worin R⁷⁰ und R⁷¹ -H sind, R⁷² und R⁷³ -CH₃ sind, n Zwei ist, und die Verbindung die Struktur aufweist:

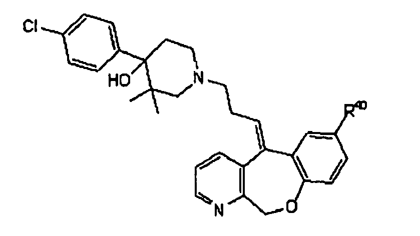

12. Verbindung nach Anspruch 11, worin R⁴⁰ ist

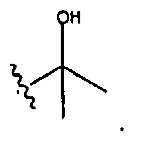

13. Verbindung mit der Struktur: 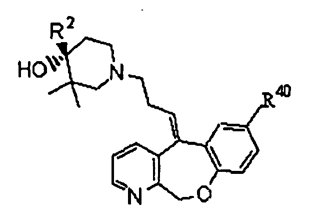 oder ein physiologisch annehmbares Salz davon, worin bedeuten:
R² ist 4-Halogenphenyl; und
R⁴⁰ ist ausgewählt aus der Gruppe bestehend aus: 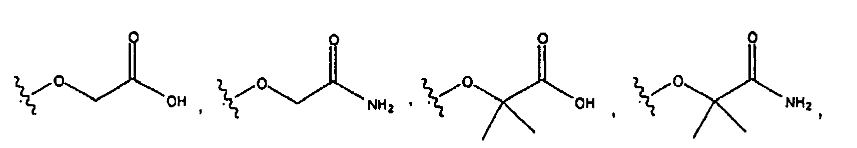 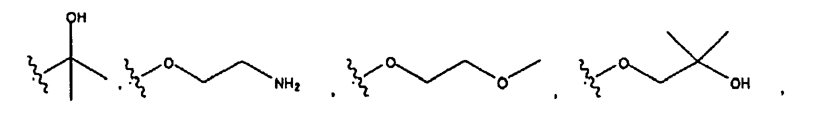 und

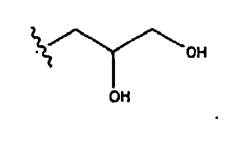

14. Verbindung nach Anspruch 13, worin R⁴⁰ ist

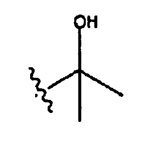

15. Verbindung nach Anspruch 13, worin R² ausgewählt ist aus der Gruppe bestehend aus 4-Chlorphenyl, 4-Bromphenyl und 4-Fluorphenyl.

16. Verbindung nach Anspruch 15, worin R² 4-Chlorphenyl ist.

17. Pharmazeutische Zusammensetzung, die eine Verbindung nach einem der Ansprüche 1 bis 16 und einen physiologisch annehmbaren Träger aufweist.

18. Verbindung nach einem der Ansprüche 1 bis 16 zur Verwendung in der Therapie.

19. Verwendung einer Verbindung nach einem der Ansprüche 1 bis 16 zur Herstellung eines Arzneimittels zur Behandlung einer Erkrankung, die assoziiert ist mit aberranter Leukozytenrekrutierung, aberranter Leukozytenaktivierung oder aberranter Leukozytenrekrutierung und -aktivierung.

20. Verwendung nach Anspruch 19, worin die Erkrankung ausgewählt ist aus der Gruppe bestehend aus Arthritis, Atherosklerose, Arteriosklerose, Restenose, Ischämie/Reperfusion-Schaden, Diabetes mellitus, Psoriasis, multiple Sklerose, entzündlichen Darmerkrankungen, Abstoßung eines transplantierten Organs oder Gewebes, Transplantat-Wirt-Erkrankung, Allergie und Asthma.

21. Verwendung einer Verbindung nach einem der Ansprüche 1 bis 16 zur Herstellung eines Arzneimittels zur Behandlung einer chronischen entzündlichen Erkrankung.

22. Verwendung einer Verbindung nach einem der Ansprüche 1 bis 16 zur Herstellung eines Arzneimittels zur Behandlung von multipler Sklerose.

23. Verwendung einer Verbindung nach einem der Ansprüche 1 bis 16 zur Herstellung eines Arzneimittels zur Behandlung von Arthritis.

24. Verwendung nach Anspruch 23, worin die Arthritis rheumatoide Arthritis ist.

## Revendications

1. Composé ayant la formule : 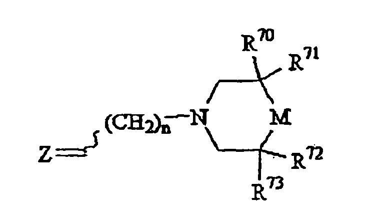 ou un sel physiologiquement acceptable de celui-ci, dans lequel :
n est compris entre un et quatre,
M est > NR² ou >CR¹R² ;
R¹ est -H, -OH, -N₃, un halogène, un groupement aliphatique, un groupement aliphatique substitué, un groupement aminoalkyle, un -O-(groupement aliphatique), un -O-(groupement aliphatique substitué), un -SH, un - S-(groupement aliphatique), un -S-(groupement aliphatique substitué), un - OC(O)-(groupement aliphatique), un -O-C(O)-(groupement aliphatique substitué), un -C(O)O-(groupement aliphatique), un -C(O)O-(groupement aliphatique substitué), un -COOH, un -CN, un -CO-NR³R⁴ ou un -NR³R⁴ ;
R² est un -OH, un halogène, un groupement acyle, un groupement acyle substitué, un -NR⁵R⁶, un groupement aliphatique, un groupement aliphatique substitué, un groupement aromatique, un groupement aromatique substitué, un groupement benzyle, un groupement benzyle substitué, un groupement hétérocyclique non-aromatique, un groupement hétérocyclique non-aromatique substitué, un -O-(groupement aromatique substitué ou non substitué), un -O-(groupement aliphatique substitué ou non substitué), un -C(O)-(groupement aromatique substitué ou non substitué) ou un -C(O)-(groupement aliphatique substitué ou non substitué) ;
R³, R⁴, R⁵ et R⁶ sont indépendamment un -H, un groupement acyle, un groupement acyle substitué, un groupement aliphatique, un groupement aliphatique substitué, un groupement aromatique, un groupement aromatique substitué, un groupement benzyle, un groupement benzyle substitué, un groupement hétérocyclique non-aromatique ou un groupement hétérocyclique non-aromatique substitué ; ou
R¹ et R², R³ et R⁴, ou R⁵ et R⁶ considérés ensemble avec l'atome sur lequel ils sont liés, forment un noyau hétérocyclique ou carbocyclique non-aromatique substitué ou non substitué ;
R⁷⁰ et R⁷¹ sont indépendamment un -H, un -OH, un -N₃, un halogène, un groupement aliphatique, un groupement aliphatique substitué, un groupement aminoalkyle, un -O-(groupement aliphatique), un -O-(groupement aliphatique substitué), un -SH, un -S-(groupement aliphatique), un -S-(groupement aliphatique substitué), un -OC(O)-(groupement aliphatique), un -O-C(O)-(groupement aliphatique substitué), un -C(O)O-(groupement aliphatique), un - C(O)O-(groupement aliphatique substitué), un -COOH, un -CN, un -CO-NR³R⁴, un -NR³R⁴, un groupement acyle, un groupement acyle substitué, un groupement benzyle, un groupement benzyle substitué, un groupement hétérocyclique non-aromatique, un groupement hétérocyclique non-aromatique substitué ou un -O-(groupement aromatique substitué ou non substitué) ;
R⁷² et R⁷³ sont indépendamment un -OH, un -N₃, un halogène, un groupement aliphatique, un groupement aliphatique substitué, un groupement aminoalkyle, un -O-(groupement aliphatique), un -O-(groupement aliphatique substitué), un -SH, un -S-(groupement aliphatique), un -S-(groupement aliphatique substitué), un -O-C(O)-(groupement aliphatique), un -O-C(O)-(groupement aliphatique substitué), un -C(O)O-(groupement aliphatique), un - C(O)O-(groupement aliphatique substitué), un -COOH, un -CN, un -CO-NR³R⁴, un -NR³R⁴, un groupement acyle, un groupement acyle substitué, un groupement benzyle, un groupement benzyle substitué, un groupement hétérocyclique non-aromatique, un groupement hétérocyclique non-aromatique substitué ou un -O-(groupement aromatique substitué ou non substitué) ;
Z est 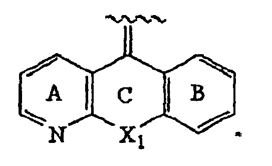 X₁ est -CH₂-O-, -O-CH₂-, -S-, -CH₂-, -CH₂-CH₂-, CH₂-S-, -S-CH₂-, -NR_{c}-CH₂-, -CH2-NR_{c}-, -SO-CH₂-, -CH₂-SO-, -S(O)₂-CH₂-, -CH₂-S(O)₂-, - CH=CH-, -NR_{c}-CO-, une liaison, -O- ou -CO-NR_{c-} ;
R_{c} est un -H, un groupement aliphatique, un groupement aliphatique substitué, un groupement aromatique, un groupement aromatique substitué, un groupement benzyle ou un groupement benzyle substitué ;
Les noyaux A et B sont indépendamment non substitués ou substitués ;
ledit groupement acyle est un groupement carbonyle aliphatique, un groupement carbonyle aromatique, un groupement sulfonyle aliphatique ou un groupement sulfonyle aromatique ;
ledit groupement aliphatique un groupement alkyle en C₁-C₆, un groupement alcényle ou un groupement alcynyle ;
ledit groupement aromatique est choisi parmi le groupe constitué par les groupements phényle, 1-naphtyle, 2-naphtyle, 1-anthracyle, 2-anthracyle, N-imidazolyle, 2-imidazolyle, 4-imidazolyle, 5-imidazolyle, 2-thiényle, 3-thiényle, 2-furanyle, 3-furanyle, 2-pyrollyle, 3-pyrrolyle, 2-pyridyle, 3-pyridyle, 4-pyridyle, 2-pyrimidyle, 4-pyrimidyle, 5-pyrimidyle, 3-pyridazinyle, 4-pyridazinyle, 3-pyrazolyle, 4-pyrazolyle, 5-pyrazolyle, 2-pyrazinyle, 2-thiazolyle, 4-thiazolyle, 5-thiazolyle, 5-tetrazolyle, 2-oxazolyle, 4-oxazolyle, 5-oxazolyle, tetrahydronaphtyle, 2-benzothiényle, 3-benzothiényle, 2-benzofuranyle, 3-benzofuranyle, 2-indolyle, 3-indolyle, 2-quinolinyle, 3-quinolinyle, 2-benzothiazolyle, 2-benzooxazolyle, 2-benzimidazolyle, 1-isoquinolinyle, 3-quinolinyle, 1-isoindolyle, 3-isoindolyle, acridinyle, 3-benzisoxazolyle, benzocyclopentyle et benzocyclohexyle ;
ledit groupement hétérocyclique non-aromatique est un cycle non-aromatique pentagonal à octogonal qui contient un ou plusieurs hétéroatomes indépendamment choisis parmi le groupe constitué par l'azote, l'oxygène et le soufre ;
ledit groupement aliphatique substitué est substitué par un ou plusieurs substituants choisis parmi le groupe constitué par le groupement oxo, le groupement epoxy, un cycle hétérocyclique non-aromatique, le groupement benzyle, le groupement benzyle substitué, le groupement aromatique ou le groupement aromatique substitué, le groupement électro-attracteur l'électron, les groupements halo, azido, -CN, -CONR²⁴R²⁵, -NR²⁴R²⁵, -OS(O)₂NR²⁴R²⁵, - S(O)₂NR²⁴R²⁵, -SO₃H, guanido, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(groupement aliphatique), -Q-(goupement aliphatique substitué), -Q-(aryle), -Q-(groupement aromatique), -Q-(groupement aromatique substitué), -Q-(CH₂)ₚ-(groupement aromatique substitué ou non substitué), -Q-(groupement hétérocylique non-aromatique) et -Q-(CH₂)ₚ-(groupement hétérocyclique non-aromatique) ;
ledit cycle hétérocyclique non-aromatique substitué est substitué par un ou plusieurs substituant choisis parmi le groupe constitué par =O, =S, un groupement électro-attracteur, les groupement halo, azido, -CN, -CONR²⁴R²⁵, - NR²⁴R²⁵, -OS(O)₂NR²⁴R²⁵, -S(O)₂NR²⁴R²⁵, -SO₃H, guanido, oxalo, - C(=NR⁶⁰)NR²¹R²², =NR⁶⁰, -(O)ᵤ-(CH₂)ₜ-C(O)OR²⁰, -(O)ᵤ(CH₂)ₜ-OC(O)OR²⁰, -(O)ᵤ(CH₂)ₜ-C(O)-NR²¹R²², -(O)ᵤ(CH₂)ₜ-NHC(O)O-R²⁰, -Q-H, -Q-(groupement aliphatique), -Q-(groupement aliphatique substitué), -Q-(aryle), -Q-(groupement aromatique), -Q-(groupement aromatique substitué), -Q-(CH₂)ₚ-(groupement aromatique substitué ou non substitué), -Q-(groupement hétérocyclique non aromatique) et -Q-(CH₂)ₚ-(groupement hétérocyclique non-aromatique)
ledit groupement aromatique substitué, groupement benzyle substitué, Cycle A lorsque substitué et Cycle B lorsque substitué, sont substitués par un ou plusieurs substituants choisis parmi le groupe constitué par le groupement électro-attracteur, le groupement aliphatique, le groupement aliphatique substitué, le groupement aromatique, le groupement aromatique substitué, les groupements halo, azido, -CN, -CONR²⁴R²⁵, -NR²⁴R²⁵, -OS(O)₂NR²⁴R²⁵, -S(O)₂NR²⁴R²⁵, - SO₃H, guanido, oxalo, -C(=NR⁶⁰)NR²¹R²², =NR⁶⁰, -(O)ᵤ-(CH₂)ₜ-C(O)OR²⁰, -(O)ᵤ(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(groupement aliphatique), -Q-(groupement aliphatique substitué), -Q-(aryle), -Q-(groupement aromatique), -Q-(groupement aromatique substitué), -Q-(CH₂)ₚ-(groupement aromatique substitué ou non substitué), -Q-(groupement hétérocyclique non-aromatique) et -Q-(CH₂)ₚ-(groupement hétérocyclique non-aromatique) ;
Q est -O-, -S-, -S(O)-, -S(O)₂-, -OS(O)₂, -C(O)-, -OC(O)-, -C(O)O-,-C(O)C(O)-O-, -O-C(O)C(O)-, -NHC(O)-, -OC(O)NH-, -NH-C(O)-NH-, - S(O)₂NH-, -NHS(O)₂, -C(NR²³)NHNH-, NHNHC(NR²³)-, - NR²⁴C(O)- ou -NR²⁴S(O)₂- ;
R²⁰, R²¹ et R²² sont indépendamment un -H, un groupement aliphatique, un groupement aromatique, un groupement hétérocyclique non-aromatique, un - NHC(O)-O-(groupement aliphatique), un -NHC(O)-O-(groupement aromatique) ou un -NHC(O)-O-(groupement hétérocyclique non-aromatique) ou R²¹ et R²², considérés ensemble avec l'atome d'azote auquel ils sont liés, peuvent former un cycle hétérocyclique non-aromatique substitué ou non substitué ;
R²³ est un -H, un groupement aliphatique, un groupement benzyle, un groupement aryle ou un groupement hétérocyclique non-aromatique ;
R²⁴ et R²⁵ sont indépendamment un -H, un groupement aliphatique, un groupement aliphatique substitué, un groupement benzyle, un groupement aryle, un groupement hétérocyclique non-aromatique ou R²⁴ et R²⁵ considérés ensemble avec l'atome d'azote auquel ils sont liés peuvent former un cycle hétérocyclique non-aromatique substitué ou non substitué.
R⁶⁰ est un -H, un -OH, un -NH₂, un groupement aromatique ou un groupement aromatique substitué.
t est zéro à trois ;
u est zéro ou un ; et
p est un à cinq.

2. Composé selon la revendication 1 dans lequel le Cycle A est non substitué et B est substitué par l'atome de carbone du Cycle B qui est lié à X, dans le noyau C, et Z est représenté par la formule structurale : 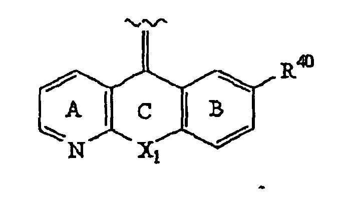 dans laquelle R⁴⁰ est -OH, -COOH, -NO₂, un halogène, un groupement aliphatique, un groupement aliphatique substitué, un groupement aromatique, un groupement aromatique substitué, -NR²⁴R²⁵, -CONR²⁴R²⁵, -NR²⁴C(O)-(groupement aliphatique) -NR²⁴C(O)-(groupement aliphatique substitué), - NR²⁴S(O)₂-(groupement aliphatique), NR²⁴S(O)₂-(groupement aliphatique substitué), -C(O)O-(groupement aliphatique), -C(O)O-(groupement aliphatique substitué), -C(O)-(groupement aliphatique), -C(O)-(groupement aliphatique substitué), -O-(groupement aliphatique), -O-(groupement aliphatique substitué), - O-(groupement aromatique), -O-(groupement aromatique substitué) et un groupement électro-attracteur, -(O)ᵤ-(CH₂)ₜC(O)OR²⁰, -(O)ᵤ-(CH₂)ₜOC(O)R²⁰, - (O)ᵤ-(CH₂)ₜC(O)-NR²¹R²² ou -(O)ᵤ-(CH₂)ₜNHC(O)O-R²⁰;
R²⁰, R²¹, R²² sont indépendamment un -H, un groupement aliphatique, un groupement aliphatique substitué, un groupement aromatique, un groupement aromatique substitué ou un groupement hétérocyclique non-aromatique ; ou
R²¹ et R²², considérés ensemble avec l'atome d'azote auquel ils sont liés, forment un cycle hétérocyclique non-aromatique ;
R²⁴ et R²⁵ sont indépendamment un -H, un groupement aliphatique ou un groupement aliphatique substitué ;
u est zéro ou un ; et
t est un nombre entier compris de zéro à 3.

3. Composé selon la revendication 2 dans lequel
M est >CR¹R² ;
R¹ est -H ou -OH ; et
R² est un groupement aromatique substitué, dans lequel ledit groupement substitué est un groupement 4-halophényle.

4. Composé selon la revendication 3 dans lequel ledit groupement 4-halophényle est choisi parmi le groupe constitué par les groupements 4-chlorophényle, 4-bromophényle et 4-fluorophényle.

5. Composé selon la revendication 3 dans lequel X₁ est -CH₂-O-.

6. Composé selon la revendication 2 dans lequel au moins un des radicaux R⁷⁰, R⁷¹, R⁷² et R⁷³ est un groupement aliphatique ou un groupement aliphatique substitué ; dans lequel -
ledit groupement aliphatique est un alkyle en C₁-C₆ et ledit groupement aliphatique substitué est un alkyle en C₁-C₆ substitué par un substituant choisi parmi le groupe constitué par un -OH, un -(O)ᵤ-(CH₂)ₜC(O)OR²⁰ et -O-(groupement aliphatique) ;
t est zéro à trois
u est zéro ou un ; et
R²⁰ est un alkyle en C₁-C₆.

7. Composé selon la revendication 6 dans lequel
R⁷⁰ et R⁷¹ sont tous deux un -H ; et
R⁷² et R⁷³ sont indépendamment choisis parmi le groupe constitué par un alkyle en C₁-C₆ et un alkyle en C₁-C₆ substitué.

8. Composé selon la revendication 7 dans lequel R⁷² est un -CH₃.

9. Composé ayant la formule : 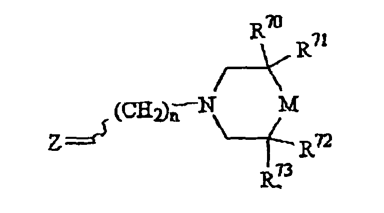 ou un sel physiologiquement acceptable de celui-ci, dans lequel :
n est un à quatre ;
M est >CR¹R² ;
R¹ est -OH ;
R² est un 4-halophényle ;
R⁷⁰ et R⁷¹ sont un -H, et R⁷² et R⁷³ sont un -CH_{3 ;} ou
R⁷⁰ et R⁷¹ sont un -CH₃, et R⁷² et R⁷³ sont un -H ;
Z est 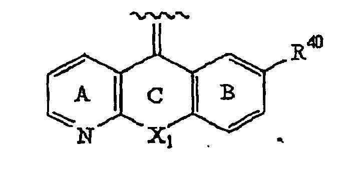 X₁ est un -CH₂-O- _{;} et
R⁴⁰ est choisi parmi le groupe constitué par :

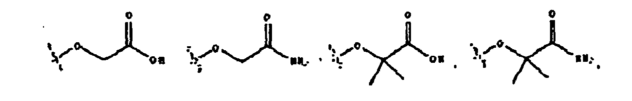

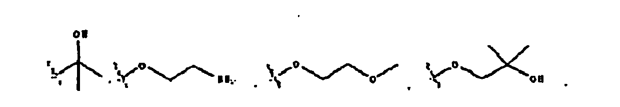

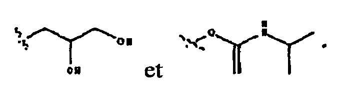

10. Composé selon la revendication 9 dans lequel ledit groupement 4-halophényle est choisi parmi le groupe constitué par les groupements 4-chlorophényle, 4-bromophényle et 4-fluorophényle.

11. Composé selon la revendication 10 dans lequel R⁷⁰ et R⁷¹ sont un - H, R⁷² et R⁷³ sont un -CH₃, n est deux et le composé a la structure suivante :

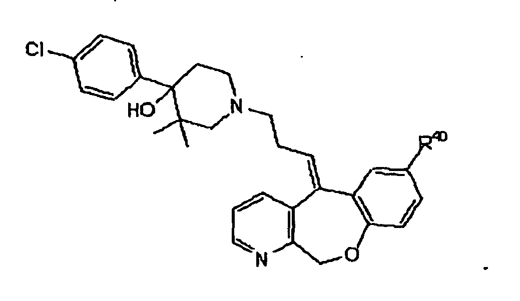

12. Composé selon la revendication 11 dans lequel R⁴⁰ est

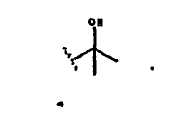

13. Composé ayant la structure : 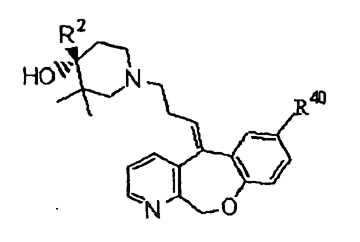 ou un sel physiologiquement acceptable de celui-ci, dans lequel
R² est un groupement 4-halophényle ; et
R⁴⁰ est choisi parmi le groupé constitué par :

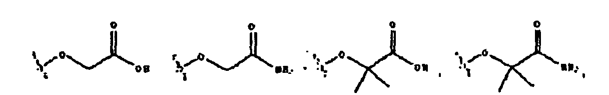

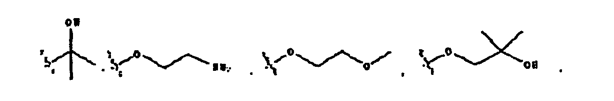

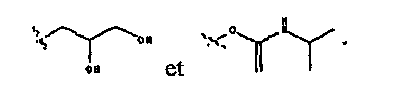

14. Composé selon la revendication 13 dans lequel R⁴⁰ est

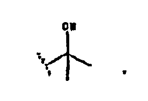

15. Composé selon la revendication 13 dans lequel R² est choisi parmi le groupe constitué par les groupements 4-chlorophényle, 4-bromophényle et 4-fluorophényle.

16. Composé selon la revendication 15 dans lequel R² est un groupement 4-chlorophényle.

17. Composition pharmaceutique comprenant un composé selon l'une quelconque des revendications 1 à 16 et un vecteur physiologiquement acceptable.

18. Composé selon l'une quelconque des revendications 1 à 16 pour utilisation en thérapie.

19. Utilisation d'un composé selon l'une quelconque des revendications 1 à 16 pour la fabrication d'un médicament pour le traitement d'une maladie associée à une mobilisation leucocytaire aberrante, une activation leucocytaire aberrante ou une mobilisation et activation leucocytaires aberrantes.

20. Utilisation de la revendication 19, dans laquelle ladite maladie est choisie parmi le groupe constitué par l'arthrite, l'athérosclérose, l'artériosclérose, la resténose, une atteinte ischémique/de reperfusion, le diabète sucré, le psoriasis, la sclérose en plaques, les infections inflammatoires du tube digestif, le rejet d'un organe ou d'un tissu greffé, la réaction du greffon contre l'hôte, les allergies et l'asthme.

21. Utilisation d'un composé selon l'une quelconque des revendications 1 à 16 pour la fabrication d'un médicament pour le traitement d'une maladie inflammatoire chronique.

22. Utilisation d'un composé selon l'une quelconque des revendications 1 à 16 pour la fabrication d'un médicament pour le traitement de la sclérose en plaques.

23. Utilisation d'un composé selon l'une quelconque des revendications 1 à 16 pour la fabrication d'un médicament pour le traitement de l'arthrite.

24. Utilisation de la revendication 23, dans laquelle ladite arthrite est l'arthrite rhumatoïde.
